# FASCICULE DE BREVET EUROPEEN

(11) **EP 2 293 770 B1**
(45) Date de publication et mention de la délivrance du brevet: **06.08.2014**
(21) Numéro de dépôt: 09769531.6
(22) Date de dépôt: 22.06.2009
(51) Int. Cl.: A61K 8/81, A61K 47/32, A61Q 1/00, A61Q 5/00, A61Q 17/04, A61Q 19/00, C08K 5/06, C08K 5/10, C08L 33/02, C08L 33/14, C08L 33/26, C08L 71/02, A61P 17/00

(54) **NOUVEAUX LATEX INVERSES SUR DES ETHERS D'ALCOOLS GRAS, COMPOSITIONS COSMETIQUES, DERMOCOSMETIQUES, DERMOPHARMACEUTIQUES OU PHARMACEUTIQUES EN COMPORTANT**
NEUE INVERSE LATEXE AUF BASIS VON FETTALKOHOLETHERN UND KOSMETISCHE, HAUTKOSMETISCHE, HAUTPHARMAZEUTISCHE ODER PHARMAZEUTISCHE ZUSAMMENSETZUNGEN DAMIT
NOVEL INVERSE LATICES BASED ON FATTY ALCOHOL ETHERS, AND COSMETIC, DERMOCOSMETIC, DERMOPHARMACEUTICAL OR PHARMACEUTICAL COMPOSITIONS COMPRISING SAME

(30) Priorité: 27.06.2008 FR 0854321
(43) Date de publication de la demande: 16.03.2011
(73) Titulaire: Société d'Exploitation de Produits pour les Industries Chimiques SEPPIC, 75007 Paris (FR)
(72) Inventeur: BRAUN, Olivier, F-81100 Castres (FR); MALLO, Paul, F-78290 Croissy-sur-Seine (FR); ROLLAND, Hervé, F-81100 Castres (FR)
(74) Mandataire: Conan, Philippe Claude
(86) Numéro de dépôt international: PCT/FR2009/051189
(87) Numéro de publication internationale: WO 2009/156690

(56) Documents cités:
- EP-A1- 1 166 771
- FR-A1- 2 903 005

## Description

La présente demande de brevet concerne des latex inverses eau dans huile, leur procédé de préparation et leur application en tant qu'épaississants et/ou émulsionnants pour des produits de soins de la peau, des cheveux et du cuir chevelu ou pour la fabrication de préparations cosmétiques, dermocosmétiques, dermopharmaceutiques ou pharmaceutiques.

Des polymères épaississants synthétiques, se présentant sous forme de latex inverses, sont décrits comme pouvant être utilisés dans la fabrication de compositions topiques, dans les demandes de brevet européen publiées sous les numéros EP 0 716 594, EP 1 047 716, EP 1 056 805, et EP 0 503 853.

Cependant, certains d'entre eux engendrent parfois des réactions d'intolérance de certaines peaux sensibles.

Pour limiter de tels effets indésirables, on a parfois essayé de remplacer les huiles minérales couramment utilisés comme phase organique de ces latex inverses, par des huiles végétales comme il l'est par exemple décrit dans la demande de brevet européen publiée sous le numéro 1 166 771. Cependant une telle utilisation provoque dans certains cas une sédimentation accélérée du latex inverse et dans d'autres cas une diminution voir le blocage de son l'inversion lorsqu'il est introduit dans une phase aqueuse.

C'est pourquoi les inventeurs se sont intéressés à la recherche de nouvelles émulsions de polymères, qui soient mieux tolérées par la peau tout en s'inversant de façon satisfaisante.

L'invention a pour objet une composition sous forme d'un latex inverse comprenant pour 100% de sa masse :
a) de 10 % massique à 80 % massique d' un polymère (P) linéaire, branché ou réticulé de l'acide 2-méthyl 2-[(1-oxo 2-propényl) amino] 1-propanesulfonique (dénommé aussi acide 2-acrylamido 2-méthyl propanesulfonique) partiellement ou totalement salifiée, et d'éventuellement un ou de plusieurs monomères choisis parmi l'acide acrylique, l'acide méthacrylique, l'acide itaconique, l'acide maléique, l'acide 3-méthyl 3-[(1-oxo 2-propènyl) amino] butanoïque, la fonction carboxylique desdits monomères étant sous forme acide, partiellement salifiés ou totalement salifiés, l'acrylamide, le méthacrylamide, le diacétoneacrylamide, le N,N-diméthyl acrylamide, le N-isopropyl acrylamide, le N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], l'acrylate de (2-hydroxy éthyle), l'acrylate de (2,3-dihydroxy propyle), le méthacrylate de (2-hydroxy éthyle), le méthacrylate de (2,3-dihydroxy propyle), un dérivé éthoxylé, de poids moléculaire compris entre 400 et 1000, de chacun de ces esters ou le vinyl pyrrolidone; les chlorures, bromures ou iodures de 2,N,N,N-tétraméthyl 2-[(1-oxo 2-propènyl) amino] propanammonium, de 2,N,N-triméthyl 2-[(1-oxo 2-propènyl) amino] propanammonium, de N,N,N-triméthyl 2-[(1-oxo 2-propènyl) oxy] éthanammonium, de N,N,N-triméthyl 3-[(1-oxo 2-propènyl) oxy] propanammonium, de N,N,N-triméthyl 2-[(1-oxo 2-propènyl) amino] propanammonium, ou de diallyl diméthyl ammonium ;
b) de 5 % massique à 10 % massique d'un système émulsionnant (S₁) de type eau dans huile (E/H),
c) de 1 % massique à 50 % massique d'eau,
d) de 5 % massique à 50 % massique d'au moins un composé choisi parmi les composés de formule (I) :

   R₁-O-R₂ (I),
dans laquelle R₁ et R₂ représente indépendamment l'un de l'autre, un radical alkyle linéaire ou ramifié comportant de 5 à 18 atomes de carbone.

Dans la composition telle que définie ci-dessus, le polymère (P) présent dans la composition objet de l'invention peut être un homopolymère ou un polymère formé à partir de plusieurs types différents de monomères. Il s'agit principalement d'un homopolymère, d'un copolymère, d'un terpolymère ou d'un tétrapolymère.

Par polymère branché, on désigne pour (P), un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne pour (P), un polymère non linéaire se présentant à l'état de réseau tridimensionnel insoluble dans l'eau, mais gonflable à l'eau et conduisant donc à l'obtention d'un gel chimique.

Selon un aspect particulier de la présente invention, dans le polymère (P) tel que défini précédemment, la proportion molaire en unité monomérique acide 2-méthyl 2-[(1-oxo 2-propènyl) amino] 1-propanesulfonique, est supérieure ou égale à 30 % et plus particulièrement supérieure ou égale à 40 %.

La composition selon l'invention peut comporter un polymère linéaire, un polymère réticulé ou un polymère branché.

Selon un aspect particulier de la présente invention, le polymère (P) est réticulé.

Lorsque le polymère (P) est réticulé, il l'est plus particulièrement avec un composé diéthylénique ou polyéthylènique dans la proportion molaire exprimée par rapport à la quantité molaire totale de monomères mis en oeuvre, inférieure ou égale à 0,25 %, et plus particulièrement inférieure ou égale à 0,05 % et tout particulièrement entre 0,005 % et 0,01 %. De préférence, l'agent de réticulation et/ou l'agent de ramification est choisi parmi le diméthacrylate d'éthylèneglycol, le diacrylate de diéthylèneglycol, le diacrylate d'éthylèneglycol, le diallyl urée, le triallylamine, le triméthylol propanetriacrylate, le méthylène-bis(acrylamide) ou un mélange de ces composés, l'acide diallyoxyacétique ou un de ses sels comme le diallyloxyacétate de sodium, ou un mélange de ces composés.

Pour les monomères à fonction acide constitutifs du polymère (P) de la composition telle que définie précédemment, le terme salifié indique qu'il s'agit de sels de métaux alcalins tels que les sels de sodium ou de potassium, les sels de bases azotées comme le sel d'ammonium, le sel de lysine ou le sel de monoéthanolamine (HO-CH₂-CH₂-NH₄⁺).

Selon un aspect particulier de la présente invention, par salifié, on signifie désigne que la fonction acide est salifiée sous forme de sel de sodium.

Dans la composition telle que définie ci-dessus, le système émulsionnant (S₁) de type eau dans huile (E/H) est constitué soit d'un seul tensioactif émulsionnant soit d'un mélange de tensioactifs émulsionnants, à condition que ledit mélange ait une valeur de HLB suffisamment faible pour induire des émulsions eau dans huile. Comme tensioactif émulsionnant de type eau-dans-huile, il y a par exemple les esters de sorbitan, comme l'oléate de sorbitan, comme celui commercialisé par la société SEPPIC sous le nom MONTANE™ 80, l'isostéarate de sorbitan, comme celui commercialisé par la société SEPPIC sous le nom MONTANE™ 70 ou le sesquioléate de sorbitan comme celui commercialisé par la société SEPPIC sous le nom MONTANE™ 83. Il y aussi certains esters de sorbitan polyéthoxylés, par exemple le monoléate de sorbitan pentaéthoxylé comme celui commercialisé par la société SEPPIC sous le nom MONTANOX™ 81 ou l'isostéarate de sorbitan pentaéthoxylé comme celui commercialisé sous le nom MONTANOX™ 71 par la société SEPPIC. Il y a encore l'alcool oléocétylique diéthoxylé comme celui commercialisé sous le nom SIMULSOL™ OC 72 par la société SEPPIC, les polyesters de poids moléculaire compris entre 1000 et 3000, produits issus de la condensation entre un acide poly(isobutényl) succinique ou son anhydride, tels que l'HYPERMER™ 2296 commercialisé par la société UNIQEMA ou encore le polyhydroxystéarate de polyglycérol (DEHYMULS™ PGPH commercialisé par la société COGNIS) ou enfin les copolymères blocks de poids moléculaire compris entre 2500 et 3500, comme l'HYPERMER™ B246 commercialisé par la société UNIQEMA ou le SIMALINE™ IE 200 commercialisé par la société SEPPIC.

Selon un aspect particulier de la composition telle que définie ci-dessus, celle-ci comprend en outre :
e) jusqu'à 5% massique d'un système émulsionnant (S₂) de type huile dans eau (H/E)

Le système émulsionnant (S₂) de type huile dans eau (H/E) mis en oeuvre dans l'aspect particulier de la composition telle que définie précédemment, est constitué soit d'un seul tensioactif émulsionnant soit d'un mélange de tensioactifs émulsionnants, à condition que ledit mélange ait une valeur de HLB suffisamment élevée pour fournir des émulsions huile dans l'eau. Comme tensioactif émulsionnant de type huile dans eau, il y a par exemple les esters de sorbitan éthoxylés comme l'oléate de sorbitan polyéthoxylé avec 20 moles d'oxyde d'éthylène, commercialisé par la société SEPPIC sous le nom de MONTANOX™ 80, le laurate de sorbitan polyéthoxylé avec 20 moles d'oxyde d'éthylène, commercialisé par la société SEPPIC sous le nom de MONTANOX™ 20, le stéarate de sorbitan polyéthoxylé avec 20 moles d'oxyde d'éthylène, commercialisé par la société SEPPIC sous le nom MONTANOX™ 60, l'huile de ricin polyéthoxylée avec 40 moles d'oxyde d'éthylène commercialisé sous le nom SIMULSOL™ OL50, l'alcool oléodécylique décaéthoxylé, commercialisé par la société SEPPIC sous le nom SIMULSOL™ OC 710, l'alcool laurique heptaéthoxylé commercialisé par la société SEPPIC sous le nom SIMULSOL™ P7, le nonylphénol décaéthoxylé commercialisé par la société SEPPIC sous le nom NONAROX™-1030 ou les hexaoléates de sorbitan polyéthoxylés commercialisés par la société SEPPIC sous le nom SIMALINE™-IE 400.

Comme autres tensioactifs émulsionnants de type huile dans eau, il y a aussi les composés de formule (II) :

R₄-O-(G)ₓ-H (II)

dans laquelle R₄ représente un radical hydrocarboné aliphatique linéaire ou ramifié, saturé ou insaturé comprenant de 1 à 30 atomes de carbones, G représente le reste du glucose ou le reste du xylose et x représente un nombre décimal compris entre 1,05 et 2,5.

La structure oligomérique (G)ₓ peut se présenter sous toute forme d'isomérie, qu'il s'agisse d'isomérie optique, d'isomérie géométrique ou d'isomérie de position ; elle peut aussi représenter un mélange d'isomères. Dans la formule (II) telle que définie précédemment, le radical R₄-O- est lié à G, par le carbone anomérique de manière à former une fonction acétal. Le nombre x, qui représente dans la formule (II), le degré moyen de polymérisation du saccharide, est plus particulièrement compris entre 1,1 et 2,0. Comme tensioactifs émulsionnants de type huile dans eau, il y a plus particulièrement, les composés de formule (II) telle que définie précédemment dans laquelle R₄ représente un radical comportant de 8 à 18 atomes de carbone et tout particulièrement dans laquelle R₄ représente plus particulièrement un radical octyle, décyle, undécyle, dodécyle, tétradécyle ou hexadécyle, lesdits radicaux étant linéaires ou ramifiés. Comme exemples de produits commerciaux contenant lesdits composés de formule (II), il y a par exemple :

Le SIMULSOL™ SL8, commercialisé par la société SEPPIC, qui est une solution aqueuse contenant pour 100% de sa masse totale entre environ 58% et 62% massique d'un mélange d'alkyl polyglycosides consistant en, pour 100% de s a masse totale, entre 45% massique et 55% en massique d'un composé de formule (II), dans laquelle G représente le reste du glucose, x est égal à environ 1,45, n est égal à 0 et R₄ représente un radical décyle, et entre 45% massique et 55% massique d'un composé de formule (II), dans laquelle G représente le reste du glucose, x est égal à environ 1,45, n est égal à 0 et R₄ représente un radical octyle ;

Le SIMULSOL™ SL10, commercialisé par la société SEPPIC, qui est une solution aqueuse contenant pour 100% de sa masse totale entre environ 53% massique et 57% massique d'un mélange d'alkyl polyglycosides, consistant en, pour 100% de sa masse totale, environ 85% massique d'un composé de formule (II), dans laquelle G représente le reste du glucose, x est égal à environ 1,45, n est égal à 0 et R₄ représente un radical décyle, environ 7,5% massique d'un composé de formule (II), dans laquelle G représente le reste du glucose, x est égal à environ 1,45, n est égal à 0 et R₄ représente un radical dodécyle et environ 7,5% massique d'un composé de formule (II), dans laquelle G représente le reste du glucose, x est égal à environ 1,45, n est égal à 0 et R₄ représente un radical tétradécyle ;

Le SIMULSOL™ SLI W, commercialisé par la société SEPPIC, qui est une solution aqueuse contenant, pour 100% de sa masse totale, entre environ 53% massique et 57% massique d'un mélange d'alkyl polyglycosides de formule (II), dans laquelle G représente le reste du glucose, x est égal à environ 1,45, n est égal à 0 et R₄ représente un mélange de radicaux isodécyle, iso-undécyle et isododécyle; ou

Le SIMULSOL™ SL26, commercialisé par la société SEPPIC, qui est une solution aqueuse contenant pour 100% de sa masse totale entre environ 50% massique et 55% massique d'un mélange d'alkyl polyglycosides consistant en, pour 100% de sa masse totale, environ 70% massique d'un composé de formule (II), dans laquelle G représente le reste du glucose, x est égal à environ 1,45, n est égal à 0 et R₄ représente un radical dodécyle, environ 25% massique d'un composé de formule (II), dans laquelle G représente le reste du glucose, x est égal à environ 1,45, n est égal à 0 et R₄ représente un radical tétradécyle et environ 5% massique d'un composé de formule (II), dans laquelle G représente le reste du glucose, x est égal à environ 1,45, n est égal à 0 et R₄ représente un radical hexadécyle.

Comme autres tensioactifs émulsionnants de type huile dans eau, il y a aussi les composés de formule (III) :

R₃-(C=O)-[O-CH₂-CH(OH)-CH₂]ₚ-OH (III)

dans laquelle R₃ représente un radical aliphatique linéaire ou ramifié comportant de 5 à 17 atomes de carbone et p un nombre entier supérieur ou égal à 3 et inférieur ou égal à 20

Comme exemples particuliers de tels composés, il y a ceux de formule (IIIa) correspondant à la formule (III) telle que définie ci-dessus dans laquelle R₃ représente un radical dans laquelle R₃ représente un radical aliphatique linéaire ou ramifié comportant de 11 à 17 atomes de carbone..

Comme autres exemples particulier de tels composés, il y a ceux de formule (IIIb) correspondant à la formule (III) telle que définie ci-dessus dans laquelle p est égal à 10.

Comme exemples de produits commerciaux contenant lesdits composés de formule (III), (IIIa) et/ou (IIIb), il y a par exemple :
le POLYADO™ 10-1-O KFG commercialisé par la société LONZA constitué essentiellement de mono-oléate de décaglycérol ;
le NIKKOL™ DECAGLYN™1-IS commercialisé par la société NIKKO Chemicals, constitué essentiellement de mono-isostéarate de décaglycérol ;
le NIKKOL™ DECAGLYN™1-L commercialisé par la société NIKKO Chemicals, constitué essentiellement de monolaurate de décaglycérol ;
le NIKKOL™ DECAGLYN™1-LN commercialisé par la société NIKKO Chemicals, constitué essentiellement de monolinoléate de décaglycérol ;
le NIKKOL™ DECAGLYN™1-M commercialisé par la société NIKKO Chemicals, constitué essentiellement de monomyristate de décaglycérol ;
le DREWPOL™ 10-1 CCK commercialisé par la société STEPAN, constitué essentiellement de monocaprylate de décaglycérol.

Selon un aspect particulier de la présente invention, le latex inverse comprend en outre jusqu'à 30% massique d'agents co-surfactants tels que par exemple le laurate de sorbitan.

Selon un autre aspect particulier la composition telle que définie précédemment comprend de 20% massique à 70% massique dudit polymère et plus particulièrement de 30% massique à 50% massique.

Selon un autre aspect particulier de la composition telle que définie précédemment dans la formule (I), R₁ et R₂ représentent notamment, indépendamment les uns des autres, un radical alkyle choisi parmi les radicaux pentyle, hexyle, 1,3-diméthyl butyle, heptyle, octyle, nonyle, isononyle, décyle, undécyle, dodécyle, tétradécyle, hexadécyle ou octadécyle.

Selon un aspect plus particulier de la composition telle que définie précédemment le composé de formule (I) est choisis parmi les composés suivants :
Le dioctyl éther, le didécyl éther, le didodécyl éther, le dodécyl octyl éther, le di-hexadécyl éther, (1,3-diméthyl butyl) tétradécyl éther, (1,3-diméthyl butyl) hexadécyl éther, le bis(1,3-diméthyl butyl) éther, le dihexyl éther.

Les éthers constitutifs de la phase huile du latex inverse objet de la présente invention sont disponibles dans le commerce ou peuvent être préparés par des méthodes connues de l'homme du métier, telles que par exemple la méthode décrite dans le brevet européen publié sous le numéro EP 0 753 500 B1.

Selon un autre aspect particulier de la présente invention, le polymère (P) compris dans le latex inverse tel que défini précédemment, est choisi parmi :
- un homopolymère de l'acide 2-méthyl 2-[(1-oxo 2-propènyl) amino] 1-propanesulfonique partiellement ou totalement salifié sous forme de sel de sodium ou de sel d'ammonium ;
- un copolymère de l'acide 2-méthyl 2-[(1-oxo 2-propènyl) amino] 1-propanesulfonique, partiellement ou totalement salifié sous forme de sel de sodium ou de sel d'ammonium, et d'acrylate de (2-hydroxy éthyle) ;
- un copolymère de l'acide 2-méthyl 2-[(1-oxo 2-propènyl) amino] 1-propanesulfonique et de l'acide acrylique partiellement ou totalement salifiés sous forme de sel de sodium ou de sel d'ammonium ;
- un copolymère de l'acide 2-méthyl 2-[(1-oxo 2-propènyl) amino] 1-propanesulfonique partiellement ou totalement salifié sous forme de sel de sodium ou de sel d'ammonium, et de l'acrylamide ;
- un copolymère de l'acide 2-méthyl 2-[(1-oxo 2-propènyl) amino] 1-propanesulfonique, partiellement ou totalement salifié sous forme de sel de sodium ou de sel d'ammonium et de N,N-diméthyl acrylamide ;
- un terpolymère de l'acide 2-méthyl 2-[(1-oxo 2-propènyl) amino] 1-propanesulfonique partiellement ou totalement salifié sous forme de sel de sodium ou de sel d'ammonium, de l'acrylamide et du vinyl pyrrolidone ;
- un terpolymère de l'acide 2-méthyl 2-[(1-oxo 2-propènyl) amino] 1-propanesulfonique partiellement ou totalement salifié sous forme de sel sodium ou de sel d'ammonium, de l'acide acrylique partiellement ou totalement salifié sous forme de sel sodium ou de sel d'ammonium et de l'acrylamide ;
- un terpolymère de l'acide 2-méthyl 2-[(1-oxo 2-propènyl) amino] 1-propanesulfonique partiellement ou totalement salifié sous forme de sel sodium ou de sel d'ammonium, de l'acide acrylique partiellement ou totalement salifié sous forme de sel sodium ou de sel d'ammonium et de N,N-diméthyl acrylamide ;
- un tétrapolymère de l'acide 2-méthyl 2-[(1-oxo 2-propènyl) amino] 1-propanesulfonique partiellement ou totalement salifié sous forme de sel sodium ou de sel d'ammonium, de l'acrylate de (2-hydroxy éthyle), de l'acide acrylique partiellement ou totalement salifié sous forme de sel sodium ou de sel d'ammonium et de N-[2-hydroxy-1,1-bis(hydroxyméthyl) éthyl] propénamide.

Selon un aspect plus particulier de la présente invention, le polymère (P) est choisi parmi :
- un copolymère réticulé de l'acide 2-méthyl 2-[(1-oxo 2-propènyl) amino] 1-propanesulfonique (a), partiellement ou totalement salifié sous forme de sel de sodium et d'acrylate de (2-hydroxy éthyle) (b) dans un rapport molaire (a) /(b) compris entre 30/90 et 90/10, et plus particulièrement compris entre 40/60 et 90/10 ;
- un copolymère réticulé de l'acide 2-méthyl 2-[(1-oxo 2-propènyl) amino] 1-propanesulfonique (a) partiellement ou totalement salifié sous forme de sel de sodium et d'acide acrylique (c) partiellement ou totalement salifié sous forme de sel de sodium dans un rapport molaire (a)/(c) supérieur à 30/70 et 90/10, et plus particulièrement compris entre 40/60 et 90/10 et tout particulièrement supérieur à 55/45 ;
- un copolymère réticulé de l'acide 2-méthyl 2-[(1-oxo 2-propènyl) amino] 1-propanesulfonique (a), partiellement ou totalement salifié sous forme de sel de sodium et d'acrylamide (d), dans un rapport molaire (a)/(d) supérieur à 30/70 et 90/10, et plus particulièrement compris entre 40/60 et 90/10 et tout particulièrement supérieur ou égal à 50/50 ;
- un copolymère réticulé de l'acide 2-méthyl 2-[(1-oxo 2-propènyl) amino] 1-propanesulfonique (a), partiellement ou totalement salifié sous forme de sel de sodium et de N,N-diméthyl acrylamide (e), dans un rapport molaire (a)/(e) supérieur à 30/70 et 90/10, et plus particulièrement compris entre 40/60 et 90/10 et tout particulièrement supérieur ou égal à 50/50 ;
- un terpolymère réticulé de l'acide 2-méthyl 2-[(1-oxo 2-propènyl) amino] 1-propanesulfonique (a) partiellement ou totalement salifié sous forme de sel de sodium, de l'acrylamide (d) et du vinyl pyrrolidone (f) dans un rapport molaire (a)/[(d)+(f)] supérieur à 30/70 et 90/10, et plus particulièrement compris entre 40/60 et 90/10 et tout particulièrement supérieur ou égal à 50/50 ;
- un terpolymère réticulé de l'acide 2-méthyl 2-[(1-oxo 2-propènyl) amino] 1-propanesulfonique (a) partiellement ou totalement salifié sous forme de sel sodium, de l'acide acrylique (c) partiellement ou totalement salifié sous forme de sel sodium et de l'acrylamide (d) ; dans un rapport molaire
   (a)/[(c) +(d)] supérieur à 30/70 et 90/10, et plus particulièrement compris entre 40/60 et 90/10 et tout particulièrement supérieur à 55/45 ;
- un terpolymère réticulé de l'acide 2-méthyl 2-[(1-oxo 2-propènyl) amino] 1-propanesulfonique (a) partiellement ou totalement salifié sous forme de sel sodium, de l'acide acrylique (c), partiellement ou totalement salifié sous forme de sel sodium et de N,N-diméthyl acrylamide (e) dans un rapport molaire (a)/(e) supérieur à 30/70 et 90/10, et plus particulièrement compris entre 40/60 et 90/10 et tout particulièrement supérieur ou égal à 50/50 ;
- un tétrapolymère réticulé de l'acide 2-méthyl 2-[(1-oxo 2-propènyl) amino] 1-propanesulfonique (a) partiellement ou totalement salifié sous forme de sel sodium, de l'acrylate de (2-hydroxy éthyle) (b), de l'acide acrylique (c) partiellement ou totalement salifié sous forme de sel sodium et de N-[2-hydroxy-1,1-bis(hydroxyméthyl) éthyl] propénamide (g), dans un rapport molaire
   (a)/[(b) + (c) + (g)] supérieur à 30/70 et inférieur ou égal 90/10, et plus particulièrement compris entre 40/60 et 90/10 et tout particulièrement supérieur ou égal à 50/50.

Le latex inverse tel que défini ci-dessus, contient généralement de 4% à 10% en poids, d'agents émulsifiants. Généralement de 20% à 50% et plus particulièrement de 25% à 40% du poids total des émulsifiants sont du type eau dans huile et de 80% à 50% et plus particulièrement de 75 à 60% sont du type huile dans eau.

Le latex inverse tel que défini ci-dessus contient généralement de 5% à 50% et de préférence de 20% à 25% de composé de formule (I) telle que définie précédemment. Ce latex peut en outre contenir un ou plusieurs additifs choisis notamment parmi les agents complexants, les agents de transfert ou les agents limiteurs de chaînes.

Le latex inverse tel que défini ci-dessus contient généralement de 1% massique à 40 % massique d'eau.

Le latex inverse objet de la présente invention est généralement préparé par polymérisation en émulsion inverse, méthode connue de l'homme du métier.

Si nécessaire ou si désiré, l'émulsion eau dans huile de polymère obtenue à l'issue de l'étape de polymérisation, peut être concentrée pour éliminer la quantité souhaitée d'eau, additionnée dudit système émulsionnant (S₂) de type huile dans eau (H/E) et/ou dudit agent co-surfactant.

L'invention a aussi pour objet l'utilisation de la composition telle que définie précédemment pour préparer une composition topique cosmétique, dermopharmaceutique ou pharmaceutique.

Une composition topique selon l'invention, destinée à être appliquée sur la peau, sur le cheveu, sur le cuir chevelu ou sur les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un agent myorelaxant, un agent antifongique ou un agent antibactérien.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un agent antirides, un agent à visée amincissante, un agent antiradicalaire, un agent antiacnéique ou un agent antifongique.

Une composition topique selon l'invention comporte habituellement pour 100% de sa masse totale entre 0,1 % et 10 % massique de l'agent épaississant défini ci-dessus. Le pH de la composition topique est de préférence supérieur ou égal à 5.

La composition topique peut en outre comporter des composés classiquement compris dans ce type de compositions, par exemple des parfums, des conservateurs, des antioxydants, des colorants, des émollients ou des tensioactifs.

Selon encore un autre aspect, l'invention concerne l'utilisation du nouvel agent épaississant conforme à l'invention, mentionné ci-dessus, pour épaissir et émulsionner une composition topique comprenant au moins une phase aqueuse.

La composition selon l'invention est un substitut intéressant à celles vendues sous les noms SEPIGEL™ 305, SEPIGEL™ 501, SIMULGEL™ EG, SIMULGEL™ NS, SIMULGEL™ INS 100, SIMULGEL™ SMS 88, SIMULGEL™ S, SEPIPLUS 400 ,SEPIPLUS 265,SEPIPLUS Sou SIMULGEL™ 600 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL ou SIMULGEL.

Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204.

Elle est particulièrement compatible avec le MONTANOV™ 68, le MONTANOV™ 82, le MONTANOV™ 202, MONTANOV™ 14, MONTANOV™ L ou le MONTANOV™ S. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316.

Elle peut également être utilisée pour former des gels aqueux à pH acide cosmétiquement ou physiologiquement acceptable, tels que ceux décrit dans WO 93/07856 ; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL^{™} pour former des produits de traitement des cheveux comme ceux décrits dans DE 195 23596 ou en association avec d'autres polymères épaississants.

La composition selon l'invention est également compatible avec les principes actifs tels que par exemple, les agents auto-bronzants comme le dihydroxyacétone (DHA) et/ou l'érythrulose, ou les agents anti-acné ; elle peut donc être introduite dans des compositions auto-bronzantes comme celles revendiquées dans EP 0 715 845, EP 0 604249, EP 0576188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou dans WO 98/09611.

Lorsque la composition telle que définie précédemment est destinée au traitement de cheveux, elle comprend plus particulièrement un latex inverse de polymère cationique objet de la présente invention.

Lorsque la composition telle que définie précédemment est destinée au traitement de la peau et/ou des muqueuses, elle comprend plus particulièrement un latex inverse de polymère anionique objet de la présente invention.

Les latex inverse objet de la présente invention peuvent être utilisés comme épaississant de pâtes d'impression textile;

Les exemples qui suivent ont pour but d'illustrer la présente invention.

### A) Exemples de préparation d'une composition selon l'invention

### Exemple 1: Préparation d'un latex inverse de copolymère (AMPS, sel de Na) / HEA (90/10), réticulé au méthylène bis(acrylamide), dans le dioctyl éther (Composition 1)

**a)** - On charge dans un bécher sous agitation :
   - 632,5 g d'une solution commerciale à 55% de 2-méthyl 2-[(1-oxo 2-propènyl) amino] 1-propanesulfonate de sodium (AMPS sel de Na),
   - 19,6 g d'acrylate de (2-hydroxy éthyle) (HEA),
   - 0,45 g de diéthylènetriaminepentacétate de sodium,
   - 0,105 g de méthylène bis(acrylamide),
   - Le pH de cette solution aqueuse est égal à 4.
**b) -** On prépare une phase organique en mélangeant :
   - 240 g de dioctyl éther d'origine végétale,
   - 16,1 g d'isostéarate de sorbitan (MONTANE™ 70),
   - 13,4 g de monolaurate de sorbitan (MONTANE™ 20), et
   - 2,9 g de polyhydroxystéarate de polyglycérol (DEHYMUL™ PGPH).
**c)** - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 2,5 ml d'une solution contenant 0,64% en poids d'hydroperoxyde de cumène dans le dioctyl éther, 0,134 g de persulfate de sodium dans 5 g d'eau, puis après homogénéisation de la solution, une solution aqueuse de métabisulfite de sodium (0,8% dans l'eau) pendant environ 60 minutes à raison de 0,15 ml /minute environ et en laissant monter la température jusqu'à la température de fin de polymérisation. On maintient alors le milieu réactionnel pendant environ 90 minutes à cette température. On ajoute alors 3% de mono laurate de décaglycérol ( DEGAGLYN™ 1L). et on obtient l'émulsion eau dans huile désirée.

### Evaluation des propriétés

Viscosité dans l'eau à 3% du latex (Brookfield RVT Mobile 6, vitesse 5) :
η = 112 000 mPa.s

Viscosité à 3% du latex dans de l'eau salée (NaCl 0,1%) (Brookfield RVT Mobile 3, vitesse 5) : η = 9 580 mPa.s.

Viscosité du latex à 25°C (Brookfield RVT Mobile 3, vitesse 20) :
η = 1 800 mPa.s

### Exemple 2 : Préparation d'un latex inverse de tétranolymère (AMPS, sel de Na) / HEA /THAM / AA (84/10/4/2), réticulé au méthylène bis(acrylamide), dans le dioctyl éther (Composition 2)

**a) -** On charge dans un bécher sous agitation :
   - 627,6 g d'une solution commerciale à 55% de 2-méthyl 2-[(1-oxo 2-propènyl) amino] 1-propanesulfonate de sodium (AMPS sel de Na),
   - 20,8 g d'acrylate de (2-hydroxy éthyle) (HEA),
   - 0,45 g de diéthylènetriaminepentacétate de sodium (VERSENEX™ 80),
   - 12,6 g de tris(hydroxyméthyl) acrylamidométhane (THAM)
   - 2,6 g d'acide acrylique
   - 0,028 g de méthylène bis(acrylamide),
   - Le pH de cette solution aqueuse est égal à 4.
**b) -** On prépare une phase organique en mélangeant :
   - 160 g de dioctyl éther d'origine végétale (COSMACOL™ OE),
   - 90 g ISOPAR™ H
   - 15 g d'isostéarate de sorbitan (MONTANE™ 70),
   - 10 g de HYPERMER™ 6212, et
   - 7,4 g de BLEMMER™ PLE 200.
**c) -** La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 2,5 ml d'une solution contenant 0,64% en poids d'hydroperoxyde de cumène dans le dioctyl éther, 0,134 g de persulfate de sodium dans 5 g d'eau, puis après homogénéisation de la solution, une solution aqueuse de métabisulfite de sodium (0,8% dans l'eau) pendant environ 60 minutes à raison de 0,15 ml /minute environ et en laissant monter la température jusqu'à la température de fin de polymérisation. On maintient alors le milieu réactionnel pendant environ 90 minutes à cette température. On ajoute alors 5% de mono laurate de décaglycérol ( DECAGLYN™ 1L) et on obtient l'émulsion eau dans huile désirée.

### Evaluation des propriétés

Viscosité du latex inverse auto-inversible à 25° C (Brookfield RVT Mobile 5, vitesse 20)
η = 19 800 mPa.s

Viscosité dans l'eau à 2% du latex inverse auto-inversible (Brookfield RVT Mobile 6, vitesse 5) :
η = 61 600 mPa.s

Viscosité dans de l'eau (contenant 0,1% chlorure de sodium) à 2% du latex inverse auto-inversible (Brookfield RVT Mobile 4, vitesse 5) :
η = 26 900 mPa.s
pH à 2% : 5,5

### Exemple 3: Préparation d'un latex inverse de copolymère (AMPS, sel de Na) / HEA (90/10), réticulé au méthylène bis(acrylamide), dans le dioctyl éther (Composition 3)

On opère comme décrit à l'exemple 1 en substituant les 3% de mono laurate de décaglycérol (DECAGLYN™ 1L) par 4% de stéarate de sorbitan polyéthoxylé avec 20 moles d'oxyde d'éthylène (MONTANOX™ 60). On obtient l'émulsion eau dans huile désirée.

### Evaluation des propriétés

Viscosité dans l'eau à 3% du latex (Brookfield RVT Mobile 6, vitesse 5) :
η = 107 000 mPa.s

Viscosité à 3% du latex dans de l'eau salée (NaCl 0,1%) (Brookfield RVT Mobile 3, vitesse 5) : η = 3 480 mPa.s.

Viscosité du latex à 25°C (Brookfield RVT Mobile 4, vitesse 20) :
η = 3 480 mPa.s

### Exemple 4: Préparation d'un latex inverse de terpolymère (AMPS, sel de Na) / N,N-diméthylacrylamide (DMAM) / acide acrylique (85/10/5), réticulé au méthylène bis(acrylamide), dans le dioctyl éther (Composition 3) 7994MP

**a) -** On charge dans un bécher sous agitation :
   - 624,5 g d'une solution commerciale à 55% de 2-méthyl 2-[(1-oxo 2-propènyl) amino] 1-propanesulfonate de sodium (AMPS sel de Na),
   - 17,5 g de (N,N-diméthylacrylamide) (DMAM),
   - 0,45g de diéthylènetriaminepentacétate de sodium (VERSENEX™ 80),
   - 6,5 g d'acide acrylique
   - 0,027 g de méthylène bis(acrylamide),
   - Le pH de cette solution aqueuse est égal à 4.
**b) -** On prépare une phase organique en mélangeant :
   - 160 g de dioctyl éther d'origine végétale (COSMACOL™ OE),
   - 90 g ISOPAR™ H
   - 17 g d'isostéarate de sorbitan (MONTANE™ 70),
   - 3g d'HYPERMER™ 6212
   - 3,66g de BLEMMER™ PLE 200,
**c) -** La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 2,5 ml d'une solution contenant 0,64% en poids d'hydroperoxyde de cumène dans le dioctyl éther, 0,3 g de persulfate de sodium dans 5 g d'eau, puis après homogénéisation de la solution, une solution aqueuse de métabisulfite de sodium (0,33% dans l'eau) pendant environ 60 minutes à raison de 0,15 ml /minute environ et en laissant monter la température jusqu'à la température de fin de polymérisation. On maintient alors le milieu réactionnel pendant environ 90 minutes à cette température. On ajoute alors 5% de l'alcool laurique heptaéthoxylé (SIMULSOL™ P7) et on obtient l'émulsion eau dans huile désirée.

### Evaluation des propriétés

Viscosité du latex inverse auto-inversible à 25° C (Brookfield RVT Mobile 3, vitesse 20)
η = 610 mPa.s

Viscosité dans l'eau à 3% du latex inverse auto-inversible (Brookfield RVT Mobile 6, vitesse 5) :
η = 26 800 mPa.s

Viscosité dans de l'eau (contenant 0,1% chlorure de sodium) à 3% du latex inverse auto-inversible (Brookfield RVT Mobile 4, vitesse 5) :
η = 15 640 mPa.s

### Exemple 5: Préparation d'un latex inverse auto-inversible concentré de terpolymère (AMPS, sel de Na) / N,N-diméthviacrvlamide (DMAM) / acide acrylique (85/10/5), réticulé au méthylène bis(acrylamide), dans le dioctyl éther (Composition 3) 7994MP

On opère de la même manière qu'à l'exemple 4 précédent mais en évaporant après l'ajout de l'inverseur l'ISOPAR™ H. On obtient un latex inverse auto-inversible comprenant environ 63 % de terpolymère.

### Evaluation des propriétés

Viscosité du latex inverse auto-inversible à 25° C (Brookfield RVT Mobile 3, vitesse 20)
η = 1 100 mPa.s

Viscosité dans l'eau à 2% du latex inverse auto-inversible (Brookfield RVT Mobile 6, vitesse 5) :
η = 32 400 mPa.s

Viscosité dans de l'eau (contenant 0,1% chlorure de sodium) à 2% du latex inverse auto-inversible (Brookfield RVT Mobile 6, vitesse 5) :
η = 22 000 mPa.s

### Exemple comparatif 1: Préparation d'un latex inverse de copolymère (AMPS, sel de Na) / HEA (90/10), réticulé au méthylène bis(acrvlamide), dans le triglycéride

### C8-C10 (Composition Ibis)

**a) -** On charge dans un bécher sous agitation :
   - 632,5 g d'une solution commerciale à 55% de 2-méthyl 2-[(1-oxo 2-propènyl) amino] 1-propanesulfonate de sodium (AMPS sel de Na),
   - 19,6 g d'acrylate de (2-hydroxy éthyle) (HEA),
   - 0,45 g de diéthylènetriaminepentacétate de sodium,
   - 0,105 g de méthylène bis(acrylamide),
   - Le pH de cette solution aqueuse est égal à 4.
**b) -** On prépare une phase organique en mélangeant :
   - 240 g de triglycéride C8-C10,
   - 16,1 g d'isostéarate de sorbitan (MONTANE™ 70),
   - 13,4 g de monolaurate de sorbitan (MONTANE™ 20), et
   - 2,9 g de polyhydroxystéarate de polyglycérol (DEHYMUL™ PGPH).
**c) -** La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 2,5 ml d'une solution contenant 0,64% en poids d'hydroperoxyde de cumène dans le triglycéride C8-C10, 0,134 g de persulfate de sodium dans 5 g d'eau, puis après homogénéisation de la solution, une solution aqueuse de métabisulfite de sodium (0,8% dans l'eau) à raison de 0,15 ml /minute environ et en laissant monter la température. On observe dans les premières minutes qui suivent l'addition de l'hydroperoxyde de cumène, une forte augmentation de la température, et un épaississement du milieu réactionnel qui bloque l'agitation, accompagné par une décantation de la phase huileuse, caractérisant ainsi la prise en masse du milieu réactionnel. Le latex inverse recherché, avec le triglycéride C8-C10 comme phase huileuse, n'est donc pas obtenu.

### B) Propriétés des compositions selon l'invention

L'ensemble des latex inverse préparés comme décrit dans les exemples 1 à 5, ne provoque pas de sédimentation accélérée des émulsions épaissies par eux. D'autre part la phase huile utilisée d'in duit aucun blocage de l'inversion contrairement à ce qui se passe parfois lorsque cette phase est un ester d'acide gras.

### C) Exemples de formulations préparées avec les compositions selon l'invention

### Exemple 1 : Baume après-rasage

| FORMULE | | | |
|---|---|---|---|
| A | Composition 1 : | | 1,5 % |
| | Eau : | qsp | 100 % |
| B | MICROPEARL™ M100 : | | 5,0 % |
| | SEPICIDE™ CI : | | 0,50 % |
| | Parfum : | | 0,20 % |
| | Ethanol à 95° : | | 10,0 % |

### MODE OPERATOIRE

Ajouter B dans A.

### Exemple 2 : Soin apaisant après-soleil

| FORMULE | | |
|---|---|---|
| Mélange de lauryl aminoacides : | | 0,1 % à 5 % |
| Aspartate de magnésium et de potassium : | | 0,002 % à 0,5 % |
| LANOL™ 99 : | | 10,0 % |
| Eau : | q.s.p. | 100 % |
| Composition 1 : | | 2,50 % |
| SEPICIDE™ HB : | | 0,3 % |
| SEPICIDE™ CI : | | 0,2 % |
| Parfum : | | 0,4 % |
| Colorant : | | 0,03 % |

### Exemple 3 : Fond de teint hydratant et matifiant

| FORMULE | | | |
|---|---|---|---|
| A | eau : | | 20,0% |
| | Butylène glycol : | | 4,0% |
| | PEG-400 : | | 4,0% |
| | PECOSIL™ PS100 : | | 1,0% |
| | Hydroxyde de sodium : | | q.s. pH = 9 |
| | Dioxyde de titane : | | 7,0% |
| | Talc : | | 2,0% |
| | Oxyde de fer jaune : | | 0,8% |
| | Oxyde de fer rouge : | | 0,3% |
| | Oxyde de fer noir : | | 0,05% |
| B | LANOL™ 99 : | | 8% |
| | Caprylic capric triglycéride | | 8% |
| | MONTANOV™ 202 : | | 5,00% |
| C | eau : | q.s.p. | 100% |
| | MICROPEARL™ M305 : | | 2,0% |
| | EDTA tétrasodé : | | 0,05% |
| D | DOW CORNING™ 345 : | | 4,0% |
| | KETROL™ T : | | 0,2% |
| | Composition 1 : | | 0,8% |
| E | SEPICIDE™ HB : | | 0,5% |
| | SEPICIDE CI : | | 03% |
| | Parfum : | | 0,2% |

### MODE OPERATOIRE

préparer à 80°C, les mélanges B + D et A + C, puis mélanger et émulsionner l'ensemble.

### Exemple 4 : Gel coup d'éclat

| FORMULE | | | |
|---|---|---|---|
| A | Composition 1 : | | 4% |
| | Eau : | | 30% |
| B | ELASTINE HPM : | | 5,0% |
| C | MICROPEARL™ M 100 : | | 3% |
| | Eau : | | 5% |
| D | SEPICIDE™ CI : | | 0,2% |
| | SEPICIDE™ HB : | | 0,3% |
| | Parfum : | | 0,06% |
| | Sodium pyrolidinonecarboxylate 50% : | | 1% |
| | Eau : | q. s. p. | 100% |

### MODE OPERATOIRE

Préparer A ; additionner B, puis C, puis D.

### Exemple 5 : Lait corporel

| FORMULE | | |
|---|---|---|
| MONTANOV™ S : | | 3,5% |
| LANOL™ 37T : | | 8,0% |
| SOLAGUM™ L : | | 0,05% |
| Eau : | q.s.p. | 100% |
| EUSOLEX™ 4360 : | | 2,0% |
| diméthicone 350cPs : | | 0,05% |
| Composition 4 : | | 0,8% |
| conservateur : | | 0,2% |
| parfum: | | 0,4% |

### Exemple 6 : Emulsion démaquillante à l'huile d'amandes douces

| FORMULE | | |
|---|---|---|
| MONTANOV™ 68 : | | 5% |
| huile d'amandes douces : | | 5% |
| eau : | q.s.p. | 100% |
| Composition 3 : | | 0,3% |
| glycérine : | | 5% |
| conservateur : | | 0,2% |
| parfum : | | 0,3% |

### Exemple 7 : Crème hydratante pour peaux grasses

| FORMULE | | |
|---|---|---|
| MONTANOV™ 68 : | | 5% |
| Cétylstéaryloctanoate : | | 8% |
| octyl palmitate : | | 2% |
| eau : | q.s.p. | 100% |
| Composition 3 : | | 0,6% |
| MICROPEARL™ M100 : | | 3,0% |
| Mucopolysaccharides : | | 5% |
| SEPICIDE™ HB : | | 0,8% |
| Parfum : | | 0,3% |

### Exemple 8 : Crème aux AHA pour peaux sensibles

| | | |
|---|---|---|
| Mélange de lauryl aminoacides : | | 0,1% à 5% |
| aspartate de magnésium et de potassium : | | 0,002% à 0,5% |
| LANOL™ 99 : | | 2% |
| MONTANOV™ 68 : | | 5,0% |
| Eau : | q.s.p. | 100% |
| Composition 4 : | | 1,50% |
| acide gluconique : | | 1,50% |
| tri éthanolamine : | | 0,9% |
| SEPICIDE™ HB : | | 0,3% |
| SEPICIDE™ CI : | | 0,2% |
| Parfum : | | 0,4% |

### Exemple 9 : Soin apaisant après soleil

| | | |
|---|---|---|
| Mélange de lauryl aminoacides : | | 0,1% à 5% |
| aspartate de magnésium et de potassium : | | 0,002% à 0,5% |
| LANOL™ 99 : | | 10,0% |
| Eau : | q.s.p. | 100% |
| Composition 2 : | | 2,50% |
| SEPICIDE™ HB : | | 0,3% |
| SEPICIDE™ CI : | | 0,2% |
| Parfum : | | 0,4% |
| Colorant : | | 0,03% |

### Exemple 10 : Lait démaquillant

| | | |
|---|---|---|
| MONTANOV™ S : | | 3% |
| PRIMOL™ 352 : | | 8,0% |
| Huile d'amandes douces : | | 2% |
| Eau : | q.s.p. | 100% |
| Composition 5 : | | 0,8% |
| conservateur : | | 0,2% |

### Exemple 11 : Emulsion fluide à pH alcalin

| | | |
|---|---|---|
| MARCOL™ 82 : | | 5,0% |
| NaOH : | | 10,0% |
| Eau : | q.s.p. | 100% |
| Composition 4 : | | 1,5% |

### Exemple 12 : Fond de teint fluide

| | | |
|---|---|---|
| SIMULSOL™ 165 : | | 5,0% |
| LANOL™ 84D : | | 8,0% |
| LANOL™ 99 : | | 5,0% |
| Eau : | q.s.p. | 100% |
| Pigments et charges minérales : | | 10,0% |
| Composition 5 : | | 1,2% |
| Conservateur : | | 0,2% |
| Parfum : | | 0,4% |

### Exemple 13 : Lait solaire

| | | |
|---|---|---|
| MONTANOV™ S : | | 3,5% |
| LANOL™ 37T : | | 10,0% |
| PARSOL™ MCX : | | 5,0% |
| EUSOLEX™ 4360 : | | 2,0% |
| Eau : | q.s.p. | 100% |
| Composition 1 : | | 1,8% |
| Conservateur : | | 0,2% |
| Parfum : | | 0,4% |

### Exemple 14 : Composition de soin non rincée

| | | |
|---|---|---|
| Composition 3 : | | 1,5% |
| Parfum : | | q. s |
| Conservateur : | | q. s. |
| DOW CORNING™ X2 8360 : | | 5,0% |
| DOW CORNING™ Q2 1401 : | | 15,0% |
| Eau : | q.s.p. | 100% |

### Exemple 15 : Gel amincissant

| | | |
|---|---|---|
| Composition 6 : | | 5% |
| Ethanol : | | 30% |
| Menthol : | | 0,1% |
| Caféine : | | 2,5% |
| Extraits de ruscus : | | 2% |
| Extrait de lierre : | | 2% |
| SEPICIDE™ HB : | | 1% |
| Eau: | q. s. p. | 100% |

### Exemple 16 : Gel crème teinté ultra naturel

| FORMULE | | | |
|---|---|---|---|
| A | eau : | | 10,0% |
| | Butylène glycol : | | 4,0% |
| | PEG-400 : | | 4,0% |
| | PECOSIL™ PS100 : | | 1,5% |
| | NaOH : | q.s. | pH = 7 |
| | Dioxyde de titane : | | 2,0% |
| | Oxyde de fer jaune : | | 0,8% |
| | Oxyde de fer rouge : | | 0,3% |
| | Oxyde de fer noir : | | 0,05% |
| B | LANOL™ 99 : | | 4,0% |
| | Caprylic capric triglycéride | | 4,0% |
| | SEPIFEEL™ ONE : | | 1,0% |
| | Composition 5 : | | 3,0% |
| C | eau : | q.s.p. | 100% |
| | MICROPEARL™ M305 : | | 2,0% |
| | EDTA tétrasodé : | | 0,05% |
| | DOW CORNING™ 245 Fluid : | | 4,0% |
| D | SEPICIDE™ HB : | | 0,5% |
| | SEPICIDE CI : | | 03% |
| | Parfum : | | 0,2% |

### MODE OPERATOIRE

préparer le mélange B + C puis ajouter A puis D.

### Exemple 17 : Soin pour les peaux grasses

| FORMULE | | | |
|---|---|---|---|
| A | MICROPEARL™ M310 : | | 1,0% |
| | Composition 5 : | | 5,0% |
| | Isononanoate d'octyle : | | 4.0% |
| B | eau : | q.s.p. | 100% |
| C | SEPICONTROL™ A5 : | | 4,0% |
| | Parfum : | | 0,1% |
| | SEPICIDE™ HB : | | 0,3% |
| | SEPICIDE™ CI : | | 0,2% |
| D | CAPIGEL™ 98 : | | 0,5% |
| | Eau : | | 10% |

### Exemple 18 : Crème aux AHA

| FORMULE | | | |
|---|---|---|---|
| A | MONTANOV™ 68 : | | 5,0% |
| | LIPACIDE™ PVB: | | 1,05% |
| | LANOL™ 99 : | | 10,0% |
| B | eau : | q.s.p. | 100% |
| | Acide gluconique : | | 1,5% |
| | TEA (triéthanolamine) : | | 0,9% |
| C | Composition 4 : | | 1,5% |
| D | parfum : | | 0,4% |
| | SEPICIDE™ HB : | | 0,2% |
| | SEPICIDE™ CI : | | 0,4% |

### Exemple 19 : Autobronzant non gras pour visage et corps

| FORMULE | | | |
|---|---|---|---|
| A | LANOL™ 2681 : | | 3,0% |
| | Composition 3 : | | 2,5% |
| B | eau : | q.s.p. | 100% |
| | Dihydroxyacétone : | | 3,0% |
| C | parfum : | | 0,2% |
| | SEPICIDE™ HB : | | 0,8% |
| | NaOH (hydroxyde de sodium) : | | qs pH = 5 |

### Exemple 20 : Lait solaire au monoï de Tahiti

| FORMULE | | | |
|---|---|---|---|
| A | Monoï de Tahiti : | | 10% |
| | LIPACIDE™ PVB : | | 0,5% |
| | Composition 7 : | | 2,2% |
| B | eau : | q.s.p. | 100% |
| C | parfum : | | 0,1% |
| | SEPICIDE™ HB : | | 0,3% |
| | SEPICIDE™ CI : | | 0,1% |
| | PARSOL™ MCX : | | 4,0% |

### Exemple 21 : Soin solaire pour le visage

| FORMULE | | | |
|---|---|---|---|
| A | DC™ 1501 : | | 4,0% |
| | Composition 5 : | | 3,5% |
| B | eau : | q.s.p. | 100% |
| C | parfum : | | 0,1% |
| | SEPICIDE™ HB : | | 0,3% |
| | SEPICIDE™ CI : | | 0,21% |
| | PARSOL™ MCX : | | 5,0% |
| | Micatitane : | | 2,0% |
| | Acide lactique : | q.s.p. | pH = 6,5 |

### Exemple 22 : Emulsion bronzante sans soleil

| FORMULE | | | |
|---|---|---|---|
| A | LANOL™ 99 : | | 15% |
| | MONTANOV™ 68 : | | 5,0% |
| | PARSOL™ MCX : | | 3,0% |
| B | eau : | q.s.p. | 100% |
| | Dihydroxyacétone : | | 5,0% |
| | Phosphate monosodique : | | 0,2% |
| C | Composition 1 : | | 0,5% |
| D | parfum : | | 0,3% |
| | SEPICIDE™ HB : | | 0,8% |
| | NaOH : | | q.s. pH=5. |

### Exemple 23 : Crème de soin

| | | |
|---|---|---|
| DOW CORNING™ 345 : | | 10% |
| Composition 8 : | | 0,8% |
| MONTANOV™ 68: | | 4,5% |
| Conservateur : | | 0,65% |
| Lysine : | | 0,025% |
| EDTA (sel disodique) : | | 0,05% |
| KETROL™ T : | | 0,2% |
| Glycérine : | | 3,0% |
| Eau : | qsp | 100% |

### Exemple 24 : Crème de soin

| | | |
|---|---|---|
| DOW CORNING™ 345 : | | 10% |
| Composition 3 : | | 0,8% |
| MONTANOV™ 68: | | 4,5% |
| Perfluoropolyméthylisopropyléther : | | 0,5% |
| Conservateur : | | 0,65% |
| Lysine : | | 0,025% |
| EDTA (sel disodique) : | | 0,05% |
| PEMULEN™ TR : | | 0,2% |
| Glycérine : | | 3% |
| Eau : | qsp | 100% |

### Exemple 25 : Lait corporel

| FORMULE | | | |
|---|---|---|---|
| A | SIMULSOL™ 165 : | | 5,0% |
| | LANOL™ 1688 : | | 12,0% |
| | LANOL™ 14 M : | | 2,0% |
| | Alcool cétylique : | | 0,3% |
| | SCHERCEMOL™ OP : | | 3% |
| B | Eau : | q.s.p. | 100% |
| C | Composition 4 : | | 0,35% |
| D | SEPICIDE™ CI : | | 0,2% |
| | SEPICIDE™ HB : | | 0,5% |
| | Parfum : | | 0,20% |

### MODE OPERATOIRE

Emulsionner B dans A vers 75°C ; ajouter C vers 60°C, puis D vers 30°C

### Exemple 26 : Lait corporel

| FORMULE | | | |
|---|---|---|---|
| A | MONTANOV™ S : | | 3,0% |
| | Triheptonate de glycérol : | | 10,0% |
| B | Eau : | q.s.p. | 100% |
| C | Composition 4 : | | 1,0% |
| D | Parfum : | q.s. | |
| | Conservateur : | q.s. | |

### MODE OPERATOIRE

Fondre A à environ 75°C. Emulsionner B dans A à 75°C ; ajouter C vers 60°C, puis D.

### Exemple 27 : Baume après-rasage apaisant sans alcool

| | | |
|---|---|---|
| Mélange de lauryl aminoacides : | | 0,1% à 5% |
| Aspartate de magnésium et de potassium : | | 0,002% à 0,5% |
| LANOL™ 99 : | | 2% |
| Huile d'amandes douces : | | 0,5% |
| Eau : | q.s.p. | 100% |
| Composition 3 : | | 3% |
| SEPICIDE™ HB : | | 0,3% |
| SEPICIDE™ CI : | | 0,2% |
| Parfum : | | 0,4% |

### Exemple 28 : Emulsion satinée pour le corps

| FORMULE | | |
|---|---|---|
| A | SIMULSOL™ 165 : | 5,0 % |
| | LANOL™ 1688 : | 8,50 % |
| | Beurre de Karité : | 2 % |
| | Huile de paraffine : | 6,5 % |
| | LANOL™ 14 M : | 3 % |
| | LANOL™^{™} S : | 0,6 % |
| B | Eau : | 66,2 % |
| C | MICROPEARL™ M100 : | 5 % |
| D | Composition 5 : | 3 % |
| E | SEPICIDE™ CI : | 0,3 % |
| | SEPICIDE™ HB : | 0,5 % |
| | MONTEINE™ CA : | 1 % |
| | Parfum : | 0,20 % |
| | Acétate de vitamine E : | 0,20 % |
| | pyrolidinonecarboxylate de sodium : | 1 % (agent hydratant) |

### MODE OPERATOIRE

Ajouter C dans B, émulsionner B dans A à 70°C, puis ajouter D à 60°C puis E à 30°C.

### Exemple 29 : crème H/E

| FORMULE | | | |
|---|---|---|---|
| A | SIMULSOL™ 165 : | | 5,0 % |
| | LANOL™ 1688: | | 20,0 % |
| | LANOL™ P : | | 1,0 % |
| B | Eau : | q.s.p. | 100 % |
| C | Composition 2 : | | 2,50 % |
| D | SEPICIDE™ CI : | | 0,20 % |
| | SEPICIDE™ HB : | | 0,30 % |

### MODE OPERATOIRE

Introduire B dans A vers 75°C ; ajouter C vers 60°C, puis D vers 45°C

### Exemple 30 : gel solaire non gras

| FORMULE | | | |
|---|---|---|---|
| A | Composition 5 : | | 3,00 % |
| | Eau : | | 30 % |
| B | SEPICIDE™ C : | | 0,20 % |
| | SEPICIDE™ HB : | | 0,30 % |
| | Parfum : | | 0,10 % |
| C | Colorant : | | qs |
| | Eau : | | 30 % |
| D | MICROPEARL™ M 100 : | | 3,00 % |
| | Eau : | q.s.p. | 100 % |
| E | Huile de silicone : | | 2,0 % |
| | PARSOL™ MCX : | | 5,00 % |

### MODE OPERATOIRE

Introduire B dans A; ajouter C puis D, puis E.

### Exemple 31 : Lait solaire

| FORMULE | | | |
|---|---|---|---|
| A | MONTANOV™ S : | | 3,0 % |
| | Huile de sésame : | | 5,0 % |
| | PARSOL™ MCX : | | 5,0 % |
| | Carraghénane λ : | | 0,10 % |
| B | Eau : | q.s.p. | 100 % |
| C | Composition 3 : | | 0,80 % |
| D | Parfum : | | q.s. |
| | Conservateur : | | q.s. |

### MODE OPERATOIRE

Emulsionner B dans A à 75°C puis ajouter C vers 60°C, puis D vers 30°C et ajuster le pH si nécessaire

### Exemple 32 : Gel de massage

| FORMULE | | |
|---|---|---|
| A | Composition 2 : | 3,5 % |
| | Eau : | 20,0 % |
| B | Colorant : | 2 gouttes /100 g |
| | Eau : | q.s. |
| C | Alcool : | 10 % |
| | Menthol : | 0,10 % |
| D | Huile de silicone : | 5,0 % |

### MODE OPERATOIRE

Ajouter B dans A; puis ajouter au mélange, C puis D

### Exemple 33 : gel soin de massage

| FORMULE | | | |
|---|---|---|---|
| A | Composition 3 : | | 3,00 % |
| | Eau : | | 30 % |
| B | SEPICIDE™ CI : | | 0,20 % |
| | SEPICIDE™ HB : | | 0,30 % |
| | Parfum : | | 0,05 % |
| C | colorant : | | q.s. |
| | Eau : | q.s.p. | 100 % |
| D | MICROPEARL™ SQL : | | 5,0 % |
| | LANOL™ 1688 : | | 2 % |

### MODE OPERATOIRE

Préparer A ; additionner B, puis C, puis D.

### Exemple 34 : Baume après-rasage apaisant sans alcool

| FORMULE | | | |
|---|---|---|---|
| A | LIPACIDE™ PVB : | | 1,0 % |
| | LANOL™ 99 : | | 2,0 % |
| | Huile d'amandes douces : | | 0,5 % |
| B | Composition 1 : | | 3,5 % |
| C | Eau : | q.s.p. | 100 % |
| D | Parfum : | | 0,4 % |
| | SEPICIDE™ HB : | | 0,4 % |
| | SEPICIDE™ CI : | | 0,2 % |

### Exemple 35: Gel rafraîchissant après-rasage

| FORMULE | | | |
|---|---|---|---|
| A | LIPACIDE™ PVB : | | 0,5 % |
| | LANOL™ 99 : | | 5,0 % |
| | Composition 3 : | | 2,5 % |
| B | eau : | q.s.p. | 100 % |
| C | MICROPEARL™ LM : | | 0,5 % |
| | Parfum : | | 0,2 % |
| | SEPICIDE™ HB : | | 0,3 % |
| | SEPICIDE™ CI : | | 0,2 % |

### Exemple 36 : Gel brillance

| | | |
|---|---|---|
| Composition 7 : | | 1,5 % |
| Silicone volatile : | | 25 % |
| Propylèneglycol : | | 25 % |
| Eau déminéralisée : | | 10 % |
| Glycérine : | qsp | 100 % |

### Exemple 37 : Gel amincissant

| | | |
|---|---|---|
| Composition 6 : | | 1,5% |
| LANOL™ 99 : | | 2% |
| Caféine: | | 5% |
| Ethanol : | | 40% |
| MICROPEARL™ LM : | | 2% |
| Eau déminéralisée : | qsp | 100% |
| Conservateur parfum : | | qs |

### Exemple 38 : Lait démaquillant

| | |
|---|---|
| SIMULSOL™ 165 : | 4% |
| MONTANOV™ 202 : | 1% |
| Caprylate-caprate triglycéride : | 15% |
| PECOSIL™ DCT : | 1 % |
| Eau déminéralisée : | qs |
| CAPIGEL™ 98 : | 0,5% |
| Composition 4 | 1% |
| PROTEOL™ APL : | 2% |
| Hydroxyde de sodium : | qsp pH = 7 |

### Exemple 39 : Masque crème "rince off" restructurant pour cheveux stressés et fragilisés

| | | |
|---|---|---|
| KETROL™ T : | | 0,5% |
| PECOSIL™ SPP50 : | | 0, 75% |
| N-cocoyl aminoacides : | | 0,70% |
| Butylèneglycol : | | 3,0% |
| Composition 1 : | | 3,0% |
| MONTANOV™ 82 : | | 3,0% |
| Huile de jojoba : | | 1,0% |
| LANOL™ P : | | 6,0% |
| AMONYL™ DM : | | 1,0% |
| LANOL™ 99 : | | 5,0% |
| SEPICIDE™ HB : | | 0,3% |
| SEPICIDE™CI : | | 0,2% |
| Parfum : | | 0,2% |
| Eau : | qsp | 100% |

### Exemple 40 : Crème solaire

| | | |
|---|---|---|
| SIMULSOL™ 165 : | | 3% |
| MONTANOV™ 202 : | | 2% |
| Benzoate C12-C15: | | 8% |
| PECOSIL™ PS 100 : | | 2% |
| Diméthicone : | 2% | |
| DOW CORNING™ 345 : | | 5% |
| PARSOL™ MCX : | | 6% |
| EUSOLEX™ 4360 : | | 4% |
| Oxyde de Titane : | | 8% |
| KETROL™ T : | | 0,2% |
| Butylène glycol : | | 5% |
| Eau déminéralisée : | qsp | 100% |
| Composition 1 : | | 1,5% |
| Conservateur, parfum : | | qs |

### Exemple 41 : Gel de soin peaux mixtes

| | | |
|---|---|---|
| Composition 1 : | | 4% |
| Squalane végétal : | | 5% |
| Diméthicone : | | 1,5% |
| SEPICONTROL™ A5 : | | 4% |
| KETROL™ T : | | 0,3% |
| Eau : 1 | qsp | 100% |
| Conservateur, Parfum : | | qs. |

### Exemple 42 : Lotion capillaire

| | | |
|---|---|---|
| Butylène glycol : | | 3, 0% |
| Composition 4 : | | 3% |
| SIMULSOL™ 1293 : | | 3,0% |
| Acide lactique : | qs | pH = 6 |
| SEPICIDE™ HB : | | 0,2% |
| SEPICIDE™ CI : | | 0,3% |
| Parfum: | | 0,3% |
| Eau : | qs | 100% |

### Exemple 43 : Shampooing protecteur et relaxant

| | | |
|---|---|---|
| AMONYL™ 675 SB : | | 5,0% |
| Sodium lauryl éther sulfate à 28% : | | 35,0% |
| Composition 1 : | | 3,0% |
| SEPICIDE™ HB: | | 0,5% |
| SEPICIDE™CI : | | 0,3% |
| Hydroxyde de sodium : | | QS pH = 7,2 |
| Parfum : | | 0,3% |
| Colorant (FDC bleu 1/jaune 5) : | | QS |
| Eau : | QSP | 100% |

### Exemple 44 : Protecteur "leave-on" ; Soin anti-stress pour cheveux

| | | |
|---|---|---|
| KETROL™ T : | | 0,5% |
| Mélange de cocoyl aminoacides : | | 3,0% |
| Butylèneglycol : | | 5,0% |
| DC 1501 : | | 5,0% |
| Composition 1 : | | 4,0% |
| SEPICIDE™ HB: | | 0,5% |
| SEPICIDE™CI : | | 0,3% |
| Parfum : | | 0,3% |
| Eau : | QSP | 100 |

### Exemple 45 : Crème vitaminée

| | | |
|---|---|---|
| SIMULSOL™ 165 : | | 5% |
| MONTANOV™ 202 : | | 1% |
| Caprylic/capric triglycérides : | | 20% |
| Palmitate de vitamine A : | | 0,2% |
| Acétate de vitamine E : | | 1% |
| MICROPEARL™ M 305 : | | 1,5% |
| Composition 1 : | | 2% |
| Eau : | qsp | 100% |
| Conservateur, parfum : | | qs |

### Exemple 46 : Gel solaire

| FORMULE | |
|---|---|
| Composition 1 : | 3,00% |
| SEPICIDE™ CI : | 0,20% |
| SEPICIDE™ HB : | 0,30% |
| Parfum : | 0,10% |
| Colorant : | qs |
| silice: | 3,00% |
| Eau : | q.s.p 100% |
| Huile de silicone : | 2,0% |
| EUSOLEX™ 4360 : | 5,00% |

### Exemple 47 : Gloss lévre

| | | |
|---|---|---|
| Composition 1 : | | 1,50% |
| SCHERMOL™ TISC : | | 15,00% |
| VISTANOL™ NPGC : | | 15,00% |
| CANDURIN™ Paprika : | | 0,50% |
| MONTANOX™ 80 : | | 1,00% |
| ANTARON™ V216 : | | 0,90% |
| Arôme Abricot : | | 0,20% |
| SEPICIDE™ HB : | | 0,50% |
| C MALTIDEX™ H16322 : | qsp | 100% |

### Exemple 48 : Poudre pressée Terre de soleil

| | | |
|---|---|---|
| Composition 1 : | | 2,00% |
| LANOL™ 99 : | | 12,00% |
| SEPIWHITE™ MSH : | | 1,00% |
| Talc : | | 33,00% |
| MICROPEARL™ M310 : | | 3,00% |
| Oxyde de fer jaune : | | 0,80% |
| Oxyde de fer rouge : | | 0,30% |
| Oxyde de fer noir : | | 0,05% |
| Mica: | qs | 100% |

### Exemple 49 : Emulsion pour peaux à tendance atopique

| | | |
|---|---|---|
| ARLACEL™ P135 : | | 2,00% |
| Composition 1 : | | 1,00% |
| LANOL™ 1688 : | | 14,00% |
| PRIMOL™ 352 : | | 8,00% |
| Glycérine : | | 5,00% |
| Eau : | qsp | 100% |
| Sulfate de magnésium : | | 0,70% |
| SEPICIDE™ HB : | | 0,30% |
| SEPICIDE™ CI : | | 0,20% |
| MICROPEARL™ M310 : | | 5,00% |

### Exemple 50 : Soin solaire apaisant (eau dans silicone)

| | | |
|---|---|---|
| Composition 1 : | | 2,00% |
| DC5225C : | | 20,00% |
| DC345 : | | 10,00% |
| SEPICALM™ VG : | | 3,00% |
| Dioxyde de titane MT100VT : | | 5,00% |
| Oxyde de zinc Z cote HP1 : | | 5,00% |
| SEPICIDE™ HB : | | 0,30% |
| Parfum : | | 0,05% |
| SEPICIDE™ CI : | | 0,20% |
| Glycérine : | | 5,00% |
| Chlorure de sodium : | | 2,00% |
| Eau : | qsp | 100% |

### Exemple 51 : Soin multi-phases

| | | |
|---|---|---|
| Composition 1 : | | 3,00% |
| C12-15 alkylbenzoate : | | 25,00% |
| AQUAXYL™ : | | 3,00% |
| SEPITONIC™ M3 : | | 1,00% |
| SEPICIDE™ HB : | | 0,50% |
| SEPICIDE™ CI : | | 0,30% |
| Eau : | qsp | 100% |

Les définitions des produits commerciaux utilisés dans les exemples sont les suivantes :
AMONYL™ 675 SB est une Cocoamidopropyl hydroxy sultaïne, commercialisée par la société SEPPIC.
ANTARON™ V216 est un polymère synthétique (PVP/hexadècene copolymer) distribué par la société UNIVAR.
AQUAXYL™ est un agent hydratant commercialisé par la société SEPPIC.
ARLACEL™ P135 Non, c'est un produit commercialisé par Uniquema (CRODA aujourd'hui). L'équivalent SEPPIC est la Simaline IE 200 SF est un PEG-30 dipolyhy-droxystéarate commercialisé par la société SEPPIC.
C MALTIDEX™ H16322 est polyol (sirop de maltitol) commercialisé par la société CERESTAR.
CANDURIN PAPRIKA est un mélange de silicate de potassium et d'aluminium et d'oxyde de fer.
CAPIGEL™ 98 est un épaississant liquide à base de copolymère acrylate commercialisé pas la société SEPPIC.
DOW CORNING™ 245 Fluid est de la cyclométhicone, commercialisée par la société DOW CORNING.
DC 345 est un cyclométhicone commercialisé par la société Dow Corning.
DC 5225C est un mélange de cyclopentasiloxane et de diméthicone copolyol commercialisé par la société DOW CORNING.
DC1501 est un mélange de cyclopentasiloxane et de diméthiconol commercialisé par la société DOW CHEMICAL.
EUSOLEX™ 4360 est de la benzophénone-3 commercialisé par la société MERCK.
KETROL™ T est de la gomme de xanthane commercialisée par la société KELCO.
LANOL™ 2681 est un mélange caprylate, caprate de coprah, commercialisé par la société SEPPIC.
LANOL™ 99 est de l'isononanoate d'isononyle commercialisé par la société SEPPIC.
LANOL™ 1688 est un ester émollient à effet non gras commercialisé par la société SEPPIC.
LANOL™ 14M et le LANOL^{®} S sont des facteurs de consistance commercialisés par la société SEPPIC.
LANOL™ 37T est du triheptanoate de glycérol, commercialisé par la société SEPPIC.
LANOL™ 84D est du malate de dioctyle commercialisé par la société SEPPIC.
LANOL™ P est un additif à effet stabilisant commercialisé par la société SEPPIC.
LIPACIDE™ PVB, est un hydrolysat de protéines de blé palmitoylé, commercialisée par la société SEPPIC.
MARCOL™ 82 est une huile de paraffine commercialisée par la société ESSO.
MICROPEARL^{™} M100 est une poudre ultra fine au toucher très doux et à action matifiante commercialisée par la société MATSUMO
MICROPEARL^{™} SQL est un mélange de micro particules renfermant du squalane qui se libère sous l'action du massage; il est commercialisé par la société MATSUMO.
MICROPEARL^{™} LM est un mélange de squalane, de polyméthylméthacrylate et de menthol, commercialisé par la société SEPPIC.
MICROPEARL™ M 305 est une poudre hydrodispersible soyeuse à base de copolymère méthylméthacrylate réticulé.
MICROPEARL™ M310 est une poudre ultra fine au toucher très doux et à action matifiante commercialisée par la société MATSUMOTO.
MONTANOV™ S est un agent nacrant, commercialisé par la société SEPPIC, à base d'un mélange d'alkyl polyglucosides tels que ceux décrits dans WO 95/13863.
MONTANOV™ 202 (arachidyl glucoside, alcool arachidylique + alcool béhènylique), est une composition auto-émulsionnable telle que celles décrites dans WO 98/17610, commercialisée par la société SEPPIC.
MONTANOV™ 82 est un agent émulsionnant à base d'alcool cétéarylique et de cocoyl-glucoside.
MONTANOV^{™} 68 est une composition auto-émulsionnable à base de (cétéaryl glucoside) et d'alcool cétéarylique, telle que décrite dans WO 92/06778, commercialisée par la société SEPPIC.
MONTANOX™ 80 est de l'oléate de sorbitan polyéthoxylé avec 20 moles d'oxyde d'éthylène, commercialisé par la société SEPPIC
MONTEINE^{™} CA est un agent hydratant commercialisé par la société SEPPIC.
MT100VT est un dioxyde de titane micronisé ayant subi un traitement de surface (hydroxyde d'aluminium / acide stéarique) distribué par la société UNIPEX.
PARSOL^{™} MCX est du para-méthoxy cinnamate d'octyle commercialisé par la société GIVAUDAN.
PARSOL NOX^{™} est un filtre solaire commercialisé par la société GIVAUDAN.
PECOSIL™ PS 100 est du diméthicone copolyol phosphate commercialisé par la société PHOENIX.
PEMULEN™ TR est un polymère acrylique commercialisé par GOODRICH.
PRIMOL™ 352 Le PRIMOL™ 352 est une huile minérale commercialisée par la société EXXON.
PROTEOL™ APL est un tensioactif moussant, commercialisée par la société SEPPIC SCHERCEMOL™ OP est un ester émollient à effet non gras.
SCHERCEMOL™ TISC est un ester (citrate de tri-isostéaryle) commercialisé par la société SCHER.
SEPICALM™ VG est un actif apaisant (sodium palmitoyl proline) commercialisé par la société SEPPIC.
SEPITONIC™ M3, mélange d'aspartate de magnésium, de gluconate de zinc et de gluconate de cuivre, est un actif énergisant commercialisé par la société SEPPIC.
SEPICONTROL^{™} A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
SEPICIDE^{™} CI, imidazolidine urée, est un agent conservateur commercialisé par la société SEPPIC.
SEPICIDE^{™} HB , qui est un mélange de phénoxyéthanol, de méthylparaben, d'éthylparaben, de propylparaben et de butylparaben, est un agent conservateur commercialisé par la société SEPPIC.
SEPIFEEL™ ONE est un mélange de palmitoylproline, de palmitoyl glutamate de magnésium et de palmitoyl sarcosinate de magnésium, tel que ceux décrits dans FR 2787323.
SEPIWHITE™MSH est un actif dépigmentant (undecylenoyl phenylalanine) commercialisé par la société SEPPIC.
SIMULSOL™ 1293 est de l'huile de castor hydrogénée et éthoxylée, avec un indice d'éthoxylation égal à 40, commercialisé par la société SEPPIC.
SIMULSOL™ 165 est du stéarate de glycérol auto-émulsionnable commercialisée par la société SEPPIC.
SOLAGUM™ L est un carraghénane commercialisé par la société SEPPIC.
VISTANOL™ NPGC est un ester (néopentyl glycol dicaprate) commercialisé par la société SEWA KASEI.
Z COTE HP1 est un oxyde de zinc micronisé ayant subi un traitement de surface distribué par GATTEFOSSE.

## Revendications

1. Composition sous forme d'un latex inverse comprenant pour 100% de sa masse :
a) de 10 % massique à 80 % massique d'un polymère (P) linéaire, branché ou réticulé de l'acide 2-méthyl 2-[(1-oxo 2-propényl) amino] 1-propanesulfonique (dénommé aussi acide 2-acrylamido 2-méthyl propanesulfonique) partiellement ou totalement salifiée, et d'éventuellement un ou de plusieurs monomères choisis parmi l'acide acrylique, l'acide méthacrylique, l'acide itaconique, l'acide maléique, l'acide 3-méthyl 3-[(1-oxo 2-propènyl) amino] butanoïque les dits monomères étant libres, partiellement salifiés ou totalement salifiés, l'acrylamide, le méthacrylamide, le diacétoneacrylamide, le N,N-diméthyl acrylamide, le N-isopropyl acrylamide, le N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], l'acrylate de (2-hydroxy éthyle), l'acrylate de (2,3-dihydroxy propyle), le méthacrylate de (2-hydroxy éthyle), le méthacrylate de (2,3-dihydroxy propyle), un dérivé éthoxylé de poids moléculaire compris entre 400 et 1000, de chacun de ces esters ou le vinyl pyrrolidone; les chlorures, bromures ou iodures de 2,N,N,N-tétraméthyl 2-[(1-oxo 2-propènyl) amino] propanammonium, de 2,N,N-triméthyl 2-[(1-oxo 2-propènyl) amino] propanammonium, de N,N,N-triméthyl 2-[(1-oxo 2-propènyl) oxy] éthanammonium, de N,N,N-triméthyl 3-[(1-oxo 2-propènyl) oxy] propanammonium, de N,N,N-triméthyl 2-[(1-oxo 2-propènyl) amino] propanammonium, ou de diallyl diméthyl ammonium ;
b) de 5 % massique à 10 % massique d'un système émulsionnant (S₁) de type eau dans huile (E/H),
c) de 1 % massique à 50 % massique d'eau.
d) de 5 % massique à 50 % massique d'au moins un composé choisi parmi les composés de formule (I) :
R₁-O-R₂ (I)
dans laquelle R₁ et R₂ représente indépendamment l'un de l'autre, un radical alkyle linéaire ou ramifié comportant de 5 à 18 atomes de carbone.

2. Composition telle que définie à la revendication 1, dans laquelle le polymère (P) est réticulé.

3. Composition telle que définie à l'une quelconque des revendications 1 ou 2, **caractérisé en ce qu'**elle comprend en outre :
e) jusqu'à 5% massique d'un système émulsionnant (S₂) de type huile dans eau (H/E).

4. Composition telle que définie à l'une quelconque des revendications 1 à 3, **caractérisé en ce qu'**elle comprend de 20% massique à 70% massique, et plus particulièrement de 30% massique à 50% massique dudit polymère (P).

5. Composition telle que définie à l'une quelconque des revendications 1 à 4, caractérisé en ce le composé de formule (I) est choisis parmi le dioctyl éther, le didécyl éther, le didodécyl éther, le dodécyl octyl éther, le dihexadécyl éther, (1,3-diméthyl butyl) tétradécyl éther, (1,3-diméthyl butyl) hexadécyl éther, le bis(1,3-diméthyl butyl) éther et le dihexyl éther.

6. Composition telle que définie à la revendication 5, caractérisée en ce le composé de formule (I) est le dioctyl éther.

7. Composition telle que définie à l'une quelconque des revendications 1 à 6, **caractérisé en ce que** le polymère (P) est choisi parmi :
- un homopolymère de l'acide 2-méthyl 2-[(1-oxo 2-propènyl) amino] 1-propanesulfonique partiellement ou totalement salifié sous forme de sel de sodium ou de sel d'ammonium ;
- un copolymère de l'acide 2-méthyl 2-[(1-oxo 2-propènyl) amino] 1-propanesulfonique, partiellement ou totalement salifié sous forme de sel de sodium ou de sel d'ammonium, et d'acrylate de (2-hydroxy éthyle) ;
- un copolymère de l'acide 2-méthyl 2-[(1-oxo 2-propènyl) amino] 1-propanesulfonique et de l'acide acrylique partiellement ou totalement salifiés sous forme de sel de sodium ou de sel d'ammonium ;
- un copolymère de l'acide 2-méthyl 2-[(1-oxo 2-propènyl) amino] 1-propanesulfonique partiellement ou totalement salifié sous forme de sel de sodium ou de sel d'ammonium, et de l'acrylamide;
- un copolymère de l'acide 2-méthyl 2-[(1-oxo 2-propènyl) amino] 1-propanesulfonique, partiellement ou totalement salifié sous forme de sel de sodium ou de sel d'ammonium et de N,N-diméthyl acrylamide ;
- un terpolymère de l'acide 2-méthyl 2-[(1-oxo 2-propènyl) amino] 1-propanesulfonique partiellement ou totalement salifié sous forme de sel de sodium ou de sel d'ammonium, de l'acrylamide et du vinyl pyrrolidone ;
- un terpolymère de l'acide 2-méthyl 2-[(1-oxo 2-propènyl) amino] 1-propanesulfonique partiellement ou totalement salifié sous forme de sel sodium ou de sel d'ammonium, de l'acide acrylique partiellement ou totalement salifié sous forme de sel sodium ou de sel d'ammonium et de l'acrylamide ;
- un terpolymère de l'acide 2-méthyl 2-[(1-oxo 2-propènyl) amino] 1-propanesulfonique partiellement ou totalement salifié sous forme de sel sodium ou de sel d'ammonium, de l'acide acrylique partiellement ou totalement salifié sous forme de sel sodium ou de sel d'ammonium et de N,N-diméthyl acrylamide ;
- un tétrapolymère de l'acide 2-méthyl 2-[(1-oxo 2-propènyl) amino] 1-propanesulfonique partiellement ou totalement salifié sous forme de sel sodium ou de sel d'ammonium, de l'acrylate de (2-hydroxy éthyle), de l'acide acrylique partiellement ou totalement salifié sous forme de sel sodium ou de sel d'ammonium et de N-[2-hydroxy-1,1-bis(hydroxyméthyl) éthyl] propénamide.

8. Composition telle que définie à la revendication 7, **caractérisé en ce que** le polymère (P) est choisi parmi :
- un copolymère réticulé de l'acide 2-méthyl 2-[(1-oxo 2-propènyl) amino] 1-propanesulfonique (a), partiellement ou totalement salifié sous forme de sel de sodium et d'acrylate de (2-hydroxy éthyle) (b) dans un rapport molaire (a) /(b) compris entre 30/90 et 90/10, et plus particulièrement compris entre 40/60 et 90/10 ;
- un copolymère réticulé de l'acide 2-méthyl 2-[(1-oxo 2-propènyl) amino] 1-propanesulfonique (a) partiellement ou totalement salifié sous forme de sel de sodium et d'acide acrylique (c) partiellement ou totalement salifié sous forme de sel de sodium dans un rapport molaire (a)/(c) supérieur à 30/70 et 90/10, et plus particulièrement compris entre 40/60 et 90/10 et tout particulièrement supérieur à 55/45 ;
- un copolymère réticulé de l'acide 2-méthyl 2-[(1-oxo 2-propènyl) amino] 1-propanesulfonique (a), partiellement ou totalement salifié sous forme de sel de sodium et d'acrylamide (d), dans un rapport molaire (a)/(d) supérieur à 30/70 et 90/10, et plus particulièrement compris entre 40/60 et 90/10 et tout particulièrement supérieur ou égal à 50/50 ;
- un copolymère réticulé de l'acide 2-méthyl 2-[(1-oxo 2-propènyl) amino] 1-propanesulfonique (a), partiellement ou totalement salifié sous forme de sel de sodium et de N,N-diméthyl acrylamide (e), dans un rapport molaire (a)/(e) supérieur à 30/70 et 90/10, et plus particulièrement compris entre 40/60 et 90/10 et tout particulièrement supérieur ou égal à 50/50 ;
- un terpolymère réticulé de l'acide 2-méthyl 2-[(1-oxo 2-propènyl) amino] 1-propanesulfonique (a) partiellement ou totalement salifié sous forme de sel de sodium, de l'acrylamide (d) et du vinyl pyrrolidone (f) dans un rapport molaire (a)/[(d)+(f)] supérieur à 30/70 et 90/10, et plus particulièrement compris entre 40/60 et 90/10 et tout particulièrement supérieur ou égal à 50/50 ;
- un terpolymère réticulé de l'acide 2-méthyl 2-[(1-oxo 2-propènyl) amino] 1-propanesulfonique (a) partiellement ou totalement salifié sous forme de sel sodium, de l'acide acrylique (c) partiellement ou totalement salifié sous forme de sel sodium et de l'acrylamide (d) ; dans un rapport molaire
(a)/[(c) +(d)] supérieur à 30/70 et 90/10, et plus particulièrement compris entre 40/60 et 90/10 et tout particulièrement supérieur à 55/45 ;
- un terpolymère réticulé de l'acide 2-méthyl 2-[(1-oxo 2-propènyl) amino] 1-propanesulfonique (a) partiellement ou totalement salifié sous forme de sel sodium, de l'acide acrylique (c), partiellement ou totalement salifié sous forme de sel sodium et de N,N-diméthyl acrylamide (e) dans un rapport molaire (a)/(e) supérieur à 30/70 et 90/10, et plus particulièrement compris entre 40/60 et 90/10 et tout particulièrement supérieur ou égal à 50/50 ;
- un tétrapolymère réticulé de l'acide 2-méthyl 2-[(1-oxo 2-propènyl) amino] 1-propanesulfonique (a) partiellement ou totalement salifié sous forme de sel sodium, de l'acrylate de (2-hydroxy éthyle) (b), de l'acide acrylique (c) partiellement ou totalement salifié sous forme de sel sodium et de N-[2-hydroxy-1,1-bis(hydroxyméthyl) éthyl] propénamide (g), dans un rapport molaire
(a)/[(b) + (c) + (g)] supérieur à 30/70 et inférieur ou égal 90/10, et plus particulièrement compris entre 40/60 et 90/10 et tout particulièrement supérieur ou égal à 50/50.

9. L'utilisation de la composition telle que définie à l'une quelconque des revendications 1 à 8, pour préparer une composition topique cosmétique, dermopharmaceutique ou pharmaceutique.

10. Composition topique **caractérisée en ce qu'**elle comprend comme agent épaississant, entre 0,1 % et 10 % en poids de la composition telle que définie à l'une quelconque des revendications 1 à 8.

## Patentansprüche

1. Zusammensetzung in Form eines Inverslatexes, der für 100 % seiner Masse Folgendes beinhaltet:
a) 10 bis 80 Masse-% eines linearen, verzweigten oder vernetzten Polymers (P) von 2-Methyl-2-[(1-oxo-2-propenyl)amino]-1-propansulfonsäure (auch 2-Acrylamido-2-methylpropansulfonsäure genannt), ganz oder teilweise versalzt, und eventuell ein oder mehrere Monomere, ausgewählt aus Acrylsäure, Methacrylsäure, Itakonsäure, Maleinsäure, 3-Methyl-3-[(1-oxo-2-propenyl)amino]-butanoesäure, wobei die Monomere frei sind, ganz oder teilweise versalzt, Acrylamid, Methacrylamid, Diacetonacrylamid, N,N-Dimethylacrylamid, N-Isopropylacrylamid, N-[2-Hydroxy-1,1-bis(hydroxymethyl)ethyl]-propenamid [oder Tris(hydroxymethyl)-acrylamidomethan oder N-Tris(hydroxymethyl)-methylacrylamid, auch THAM genannt], (2-Hydroxyethyl)acrylat, (2,3-Dihydroxypropyl)acrylat, (2-Hydroxyethyl)methacrylat, (2,3-Dihydroxypropyl)methacrylat, ein ethoxyliertes Derivat mit einem Molekulargewicht zwischen 400 und 1000 von jedem dieser Ester, oder Vinylpyrrolidon; die Chloride, Bromide oder lodide von 2,N,N,N-Tetramethyl-2-[(1-oxo-2-propenyl)amino]-propanammonium, 2,N,N-Trimethyl-2-[(1-oxo-2-propenyl)amino]-propanammonium, N,N,N-Trimethyl-2-[(1-oxo-2-propenyl)oxy]-ethanammonium, N,N,N-Trimethyl-3-[(1-oxo-2-propenyl)oxy]-propanammonium, N,N,N-Trimethyl-2-[(1-oxo-2-propenyl)amino]-propanammonium, oder Diallyldimethylammonium;
b) 5 bis 10 Masse-% eines Emulgiersystems (S₁) des Wasser-in-Öl-Typs (E/H);
c) 1 bis 50 Masse-% Wasser;
d) 5 bis 50 Masse-% von wenigstens einer Verbindung, die aus den Verbindungen der folgenden Formel (I) ausgewählt sind:
R₁-O-R₂ (I)
wobei R₁ und R₂ unabhängig voneinander ein lineares oder verzweigtes Alkylradikal mit 5 bis 18 Kohlenstoffatomen repräsentieren.

2. Zusammensetzung gemäß Definition in Anspruch 1, wobei das Polymer (P) vernetzt ist.

3. Zusammensetzung gemäß Definition in Anspruch 1 oder 2, **dadurch gekennzeichnet, dass** sie darüber hinaus Folgendes umfasst:
e) bis zu 5 Masse-% eines Emulgiersystems (S₂) des Öl-in-Wasser-Typs (H/E).

4. Zusammensetzung gemäß Definition in einem der Ansprüche 1 bis 3, **dadurch gekennzeichnet, dass** sie 20 bis 70 Masse-% und spezieller 30 bis 50 Masse-% des Polymers (P) enthält.

5. Zusammensetzung gemäß Definition in einem der Ansprüche 1 bis 4, **dadurch gekennzeichnet, dass** die Verbindung der Formel (I) ausgewählt ist aus Dioctylether, Didecylether, Didodecylether, Dodecyloctylether, Dihexadecylether, (1,3-Dimethylbutyl)tetradecylether, (1,3-Dimethylbutyl)hexadecylether, Bis(1,3-dimethylbutyl)ether und Dihexylether.

6. Zusammensetzung gemäß Definition in Anspruch 5, **dadurch gekennzeichnet, dass** die Verbindung von Formel (I) Dioctylether ist.

7. Zusammensetzung gemäß Definition in einem der Ansprüche 1 bis 6, **dadurch gekennzeichnet, dass** das Polymer (P) ausgewählt ist aus:
- einem Homopolymer von 2-Methyl-2-[(1-oxo-2-propenyl)amino]-1-propansulfonsäure, ganz oder teilweise versalzt in Form von Natriumsalz oder Ammoniumsalz;
- einem Copolymer von 2-Methyl-2-[(1-oxo-2-propenyl)amino]-1-propansulfonsäure, ganz oder teilweise versalzt in Form von Natriumsalz oder Ammoniumsalz, und von (2-Hydroxyethyl)acrylat;
- einem Copolymer von 2-Methyl-2-[(1-oxo-2-propenyl)amino]-1-propansulfonsäure und Acrylsäure, ganz oder teilweise versalzt in Form von Natriumsalz oder Ammoniumsalz;
- einem Copolymer von 2-Methyl-2-[(1-oxo-2-propenyl)amino]-1-propansulfonsäure, ganz oder teilweise versalzt in Form von Natriumsalz oder Ammoniumsalz, und von Acrylamid;
- einem Copolymer von 2-Methyl-2-[(1-oxo-2-propenyl)amino]-1-propansulfonsäure, ganz oder teilweise versalzt in Form von Natriumsalz oder Ammoniumsalz, und von N,N-Dimethylacrylamid;
- einem Terpolymer von 2-Methyl-2-[(1-oxo-2-propenyl)amino]-1-propansulfonsäure, ganz oder teilweise versalzt, in Form von Natriumsalz oder Ammoniumsalz, von Acrylamid oder von Vinylpyrrolidon;
- einem Terpolymer von 2-Methyl-2-[(1-oxo-2-propenyl)amino]-1-propansulfonsäure, ganz oder teilweise versalzt in Form von Natriumsalz oder Ammoniumsalz, von Acrylsäure, ganz oder teilweise versalzt in Form von Natriumsalz oder Ammoniumsalz, und von Acrylamid;
- einem Terpolymer von 2-Methyl-2-[(1-oxo-2-propenyl)amino]-1-propansulfonsäure, ganz oder teilweise versalzt in Form von Natriumsalz oder Ammoniumsalz, von Acrylsäure, ganz oder teilweise versalzt in Form von Natriumsalz oder Ammoniumsalz, und von N,N-Dimethylacrylamid;
- einem Tetrapolymer von 2-Methyl-2-[(1-oxo-2-propenyl)amino]-1-propansulfonsäure, ganz oder teilweise versalzt in Form von Natriumsalz oder Ammoniumsalz, von (2-Hydroxyethyl)acrylat, von Acrylsäure, ganz oder teilweise versalzt in Form von Natriumsalz oder Ammoniumsalz, und von N-[2-Hydroxy-1,1-bis(hydroxymethyl)ethyl]-propenamid.

8. Zusammensetzung gemäß Definition in Anspruch 7, **dadurch gekennzeichnet, dass** das Polymer (P) ausgewählt ist aus:
- einem vernetzten Copolymer von (a) 2-Methyl-2-[(1-oxo-2-propenyl)amino]-1-propansulfonsäure, ganz oder teilweise versalzt, in Form von Natriumsalz, und (b) (2-Hydroxyethyl)acrylat, in einem Molverhältnis (a):(b) zwischen 30:90 und 90:10 und spezieller zwischen 40:60 und 90:10;
- einem vernetzten Copolymer von (a) 2-Methyl-2-[(1-oxo-2-propenyl)amino]-1-propansulfonsäure, ganz oder teilweise versalzt in Form von Natriumsalz, und (c) Acrylsäure, ganz oder teilweise versalzt in Form von Natriumsalz, in einem Molverhältnis (a):(c) von über 30:70 und 90:10 und spezieller zwischen 40:60 und 90:10 und ganz besonders über 55:45;
- einem vernetzten Copolymer von (a) 2-Methyl-2-[(1-oxo-2-propenyl)amino]-1-propansulfonsäure, ganz oder teilweise versalzt in Form von Natriumsalz, und (d) Acrylamid, in einem Molverhältnis (a):(d) von über 30:70 und 90:10 und spezieller zwischen 40:60 und 90:10 und ganz besonders gleich oder höher als 50:50;
- einem vernetzten Copolymer von (a) 2-Methyl-2-[(1-oxo-2-propenyl)amino]-1-propansulfonsäure, ganz oder teilweise versalzt in Form von Natriumsalz, und (e) N,N-Dimethylacrylamid in einem Molverhältnis (a):(e) von über 30:70 und 90:10 und spezieller zwischen 40:60 und 90:10 und ganz besonders gleich oder höher als 50:50;
- einem vernetzten Terpolymer von (a) 2-Methyl-2-[(1-oxo-2-propenyl)amino]-1-propansulfonsäure, ganz oder teilweise versalzt in Form von Natriumsalz, (d) Acrylamid und (f) Vinylpyrrolidon, in einem Molverhältnis (a):[(d)+(f)] von über 30:70 und 90:10 und spezieller zwischen 40:60 und 90:10 und ganz besonders gleich oder höher als 50:50;
- einem vernetzten Terpolymer von (a) 2-Methyl-2-[(1-oxo-2-propenyl)amino]-1-propansulfonsäure, ganz oder teilweise versalzt in Form von Natriumsalz, (c) Acrylsäure, ganz oder teilweise versalzt in Form von Natriumsalz, und (d) Ammoniumsalz; in einem Molverhältnis (a):[(c)+(d)] von über 30:70 und 90:10, spezieller zwischen 40:60 und 90:10 und ganz besonders über 55:45;
- einem vernetzten Terpolymer von (a) 2-Methyl-2-[(1-oxo-2-propenyl)amino]-1-propansulfonsäure, ganz oder teilweise versalzt in Form von Natriumsalz, (c) Acrylsäure, ganz oder teilweise versalzt in Form von Natriumsalz, und (e) N,N-Dimethylacrylamid, in einem Molverhältnis (a):(e) von über 30:70 und 90:10 und spezieller zwischen 40:60 und 90:10 und ganz besonders gleich oder höher als 50:50;
- einem vernetzten Tetrapolymer von (a) 2-Methyl-2-[(1-oxo-2-propenyl)amino]-1-propansulfonsäure, ganz oder teilweise versalzt in Form von Natriumsalz, (b) (2-Hydroxyethyl)acrylat, (c) Acrylsäure, ganz oder teilweise versalzt in Form von Natriumsalz, und (g) N-[2-Hydroxy-1,1-bis(hydroxymethyl)ethyl]-propenamid, in einem Molverhältnis (a):[(b)+(c)+(g)] von über 30:70 und gleich oder niedriger als 90:10, und spezieller zwischen 40:60 und 90:10 und ganz besonders gleich oder höher als 50:50.

9. Verwendung der Zusammensetzung gemäß Definition in einem der Ansprüche 1 bis 8 für die Herstellung einer topischen kosmetischen, dermopharmazeutischen oder pharmazeutischen Zusammensetzung.

10. Topische Zusammensetzung, **dadurch gekennzeichnet, dass** sie als Verdickungsmittel zwischen 0,1 und 10 Gew.-% der Zusammensetzung nach Definition in einem der Ansprüche 1 bis 8 enthält.

## Claims

1. Composition in the form of an inverse latex comprising, for 100 % of its mass:
a) from 10 % by mass to 80 % by mass of a linear, branched or cross-linked polymer (P) of 2-methyl-2-[(1-oxo-2-propenyl)amino]-1-propanesulfonic acid (also known as 2-acrylamido-2-methylpropanesulfonic acid) which is partially or totally salified, and optionally one or more monomers chosen from acrylic acid, methacrylic acid, itaconic acid, maleic acid or 3-methyl-3-[(1-oxo-2-propenyl)amino]butanoic acid, said monomers being free, partially salified or totally salified, acrylamide, methacrylamide, diacetone acrylamide, N,N-dimethylacrylamide, N-isopropyl-acrylamide, N-[2-hydroxy-1,1-bis(hydroxymethyl)-ethyl]propenamide [or tris(hydroxymethyl)acrylamidomethane or N-tris(hydroxymethyl)methylacrylamide also known as THAM], (2-hydroxyethyl)acrylate, (2,3-dihydroxypropyl)acrylate, (2-hydroxyethyl)methacrylate, (2,3-dihydroxypropyl)methacrylate, an ethoxylated derivative, of a molecular weight of between 400 and 1000, of each of these esters or vinylpyrrolidone; 2,N,N,N-tetramethyl-2-[(1-oxo-2-propenyl)amino]propanammonium chloride, bromide or iodide, 2,N,N-trimethyl-2-[(1-oxo-2-propenyl)amino]-propanammonium chloride, bromide or iodide, N,N,N-trimethyl-2-[(1-oxo-2-propenyl)oxy]ethanammonium chloride, bromide or iodide, N,N,N-trimethyl-3-[(1-oxo-2-propenyl)oxy]propanammonium chloride, bromide or iodide, N,N,N-trimethyl-2-[(1-oxo-2-propenyl)-amino]propanammonium chloride, bromide or iodide, or diallyldimethylammonium chloride, bromide or iodide;
b) from 5 % by mass to 10 % by mass of an emulsifying system (S₁) of water-in-oil (W/O) type,
c) from 1 % by mass to 50 % by mass of water,
d) from 5 % by mass to 50 % by mass of at least one compound chosen from the compounds of formula (I):
R₁-O-R₂ (I),
in which R₁ and R₂ represent, independently of one another, a linear or branched alkyl radical containing from 5 to 18 carbon atoms.

2. Composition according to claim 1, in which the polymer (P) is cross-linked.

3. Composition according to either claim 1 or claim 2, **characterised in that** it also comprises:
e) up to 5 % by mass of an emulsifying system (S₂) of oil-in-water (O/W) type.

4. Composition according to any of claims 1 to 3, **characterised in that** it comprises from 20 % by mass to 70 % by mass, and more particularly from 30 % by mass to 50 % by mass of said polymer (P).

5. Composition according to any of claims 1 to 4, **characterised in that** the compound of formula (I) is chosen from dioctyl ether, didecyl ether, didodecyl ether, dodecyl octyl ether, dihexadecyl ether, (1,3-dimethylbutyl) tetradecyl ether, (1,3-dimethylbutyl) hexadecyl ether, bis(1,3-dimethylbutyl)ether and dihexyl ether.

6. Composition according to claim 5, **characterised in that** the compound of formula (I) is dioctyl ether.

7. Composition according to any of claims 1 to 6, **characterised in that** the polymer (P) is chosen from:
- a homopolymer of 2-methyl-2-[(1-oxo-2-propenyl)-amino]-1-propanesulfonic acid, partially or totally salified in the form of a sodium salt or of an ammonium salt;
- a copolymer of 2-methyl-2-[(1-oxo-2-propenyl)amino]-1-propanesulfonic acid, partially or totally salified in the form of a sodium salt or an ammonium salt, and of (2-hydroxyethyl)acrylate;
- a copolymer of 2-methyl-2-[(1-oxo-2-propenyl)amino]-1-propanesulfonic acid and of acrylic acid, partially or totally salified in the form of a sodium salt or of an ammonium salt;
- a copolymer of 2-methyl-2-[(1-oxo-2-propenyl)amino]-1-propanesulfonic acid, partially or totally salified in the form of a sodium salt or of an ammonium salt, and of acrylamide;
- a copolymer of 2-methyl-2-[(1-oxo-2-propenyl)amino]-1-propanesulfonic acid, partially or totally salified in the form of a sodium salt or of an ammonium salt, and of N,N-dimethylacrylamide;
- a terpolymer of 2-methyl-2-[(1-oxo-2-propenyl)amino]-1-propanesulfonic acid, partially or totally salified in the form of a sodium salt or of an ammonium salt, of acrylamide and of vinylpyrrolidone;
- a terpolymer of 2-methyl-2-[(1-oxo-2-propenyl)amino]-1-propanesulfonic acid, partially or totally salified in the form of a sodium salt or of an ammonium salt, of acrylic acid, partially or totally salified in the form of a sodium salt or of an ammonium salt, and of acrylamide;
- a terpolymer of 2-methyl-2-[(1-oxo-2-propenyl)amino]-1-propanesulfonic acid, partially or totally salified in the form of a sodium salt or of an ammonium salt, of acrylic acid, partially or totally salified in the form of a sodium salt or of an ammonium salt, and of N,N-dimethylacrylamide;
- a tetrapolymer of 2-methyl-2-[(1-oxo-2-propenyl)-amino]-1-propanesulfonic acid, partially or totally salified in the form of a sodium salt or of an ammonium salt, of (2-hydroxyethyl)acrylate, of acrylic acid, partially or totally salified in the form of a sodium salt or of an ammonium salt, and of N-[2-hydroxy-1,1-bis(hydroxymethyl)ethyl]propenamide.

8. Composition according to claim 7, **characterised in that** the polymer (P) is chosen from:
- a cross-linked copolymer of 2-methyl-2-[(1-oxo-2-propenyl)amino]-1-propanesulfonic acid (a), partially or totally salified in the form of a sodium salt, and of (2-hydroxyethyl)acrylate (b), in an (a)/(b) molar ratio of between 30/90 and 90/10, and more particularly between 40/60 and 90/10;
- a cross-linked copolymer of 2-methyl-2-[(1-oxo-2-propenyl)amino]-1-propanesulfonic acid (a), partially or totally salified in the form of a sodium salt, and of acrylic acid (c), partially or totally salified in the form of a sodium salt, in an (a)/(c) molar ratio of greater than 30/70 and 90/10, and more particularly between 40/60 and 90/10, and most particularly greater than 55/45;
- a cross-linked copolymer of 2-methyl-2-[(1-oxo-2-propenyl)amino]-1-propanesulfonic acid (a), partially or totally salified in the form of a sodium salt, and of acrylamide (d), in an (a)/(d) molar ratio of greater than 30/70 and 90/10, and more particularly between 40/60 and 90/10, and most particularly greater than or equal to 50/50;
- a cross-linked copolymer of 2-methyl-2-[(1-oxo-2-propenyl)amino]-1-propanesulfonic acid (a), partially or totally salified in the form of a sodium salt, and of N,N-dimethylacrylamide (e), in an (a)/(e) molar ratio of greater than 30/70 and 90/10, and more particularly between 40/60 and 90/10, and most particularly greater than or equal to 50/50;
- a cross-linked terpolymer of 2-methyl-2-[(1-oxo-2-propenyl)amino]-1-propanesulfonic acid (a), partially or totally salified in the form of a sodium salt, of acrylamide (d) and of vinylpyrrolidone (f), in an (a)/[(d)+(f)] molar ratio of greater than 30/70 and 90/10, and more particularly between 40/60 and 90/10, and most particularly greater than or equal to 50/50;
- a cross-linked terpolymer of 2-methyl-2-[(1-oxo-2-propenyl)amino]-1-propanesulfonic acid (a), partially or totally salified in the form of a sodium salt, of acrylic acid (c), partially or totally salified in the form of a sodium salt, and of acrylamide (d), in an (a)/[(c)+(d)] molar ratio of greater than 30/70 and 90/10 and more particularly between 40/60 and 90/10, and most particularly greater than 55/45;
- a cross-linked terpolymer of 2-methyl-2-[(1-oxo-2-propenyl)amino]-1-propanesulfonic acid (a), partially or totally salified in the form of a sodium salt, of acrylic acid (c), partially or totally salified in the form of a sodium salt, and of N,N-dimethylacrylamide (e), in an (a)/(e) molar ratio of greater than 30/70 and 90/10, and more particularly between 40/60 and 90/10, and most particularly greater than or equal to 50/50;
- a cross-linked tetrapolymer of 2-methyl-2-[(1-oxo-2-propenyl)amino]-1-propanesulfonic acid (a), partially or totally salified in the form of a sodium salt, of (2-hydroxyethyl)acrylate (b), of acrylic acid (c), partially or totally salified in the form of a sodium salt, and of N-[2-hydroxy-1,1-bis(hydroxymethyl)-ethyl]propenamide (g), in an (a)/[(b)+(c)+(g)] molar ratio of greater than 30/70 and less than or equal to 90/10, and more particularly between 40/60 and 90/10, and most particularly greater than or equal to 50/50.

9. Use of the composition according to any of claims 1 to 8, for preparing a cosmetic, dermopharmaceutical or pharmaceutical topical composition.

10. Topical composition, **characterised in that** it comprises, as a thickener, between 0.1 % and 10 % by weight of the composition according to any of claims 1 to 8.
